(19)

Europäisches
Patentamt
European
Patent Office
Office européen
des brevets

(11)  **EP 1 937 458 B1**

(12)  **EUROPEAN PATENT SPECIFICATION**

(45) Date of publication and mention
of the grant of the patent:
**04.04.2012  Bulletin 2012/14**

(51) Int Cl.:
*B29C 65/08* (2006.01)       *D04H 13/00* (2006.01)
*B32B 37/00* (2006.01)       *B32B 23/08* (2006.01)

(21) Application number: **06814353.6**

(22) Date of filing: **08.09.2006**

(86) International application number:
**PCT/US2006/035074**

(87) International publication number:
**WO 2007/046974 (26.04.2007 Gazette 2007/17)**

(54) **METHOD FOR PRODUCING HIGH SPEED, PRESSURE BONDED, THIN SHEET LAMINATE**

VERFAHREN ZUR HERSTELLUNG EINES UNTER HOHER GESCHWINDIGKEIT UND UNTER DRUCK VERBUNDENES DÜNNSCHICHTLAMINAT

PROCÉDÉ DE FABRICATION D'UN STRATIFIÉ A FEUILLE MINCE, LIE PAR PRESSION, A GRANDE VITESSE

(84) Designated Contracting States:
**DE FR GB IT**

(30) Priority:  **20.10.2005  US 255114**

(43) Date of publication of application:
**02.07.2008  Bulletin 2008/27**

(73) Proprietor: **KIMBERLY-CLARK WORLDWIDE, INC.**
**Neenah, WI 54956 (US)**

(72) Inventors:
• **SHANNON, Thomas, Gerard**
**Neenah, WI 54956 (US)**
• **ZHOU, Peiguang**
**Appleton, WI 54915 (US)**
• **ZHUANG, Shiming**
**Menasha, WI 54952 (US)**

• **WILLIAMS, Gary, Douglas**
**Neenah, WI 54956 (US)**
• **SENAPATI, Nagabhusan**
**Appleton, WI 54913 (US)**

(74) Representative: **Chiva, Andrew Peter et al**
**Dehns**
**St Bride's House**
**10 Salisbury Square**
**London**
**EC4Y 8JD (GB)**

(56) References cited:
**EP-A2- 0 241 263       EP-A2- 0 689 930**
**WO-A-00/09314       WO-A-98/28123**
**US-A1- 2002 062 900**

## Description

### FIELD OF THE INVENTION

The present invention relates to a bonded web. More particularly, the present invention pertains to a bonded web laminate formed by inducing a high-rate of shear deformation.

### BACKGROUND OF THE INVENTION

Bonded webs containing polymeric films and fibrous layers have been produced by employing conventional bonding techniques such as adhesive bonding, thermal bonding, and ultrasonic bonding. WO 98/28123 and WO 00/09314 disclose methods of bonding two layers together using rotary ultrasonic horns. The bonded webs have been formed by delivering target webs of materials through the nip formed between a pair of counter-rotating bonding rollers, and have included bonding rollers with smooth surfaces, bonding rollers with surfaces that include distributed patterns of bonding elements, and combinations of rollers with smooth and patterned surfaces. The bonding techniques have employed combinations of heat and pressure to effect the desired bonding. In particular arrangements, the bonding rollers have been differently constructed or differently rotated to produce a surface speed differential between the bonding rollers.

Bonded webs constructed with adhesive can be produced with adequate bond strengths at high speeds, but the added cost of the adhesive has been excessive. Where the bonded web has been intended to provide an absorbent sheet article, the presence of the adhesive has also excessively inhibited the absorbent properties of the bonded web. The presence of adhesive has, for example, excessively degraded the absorbent capacity and the absorbent rate of the adhesive-bonded webs. Bonded webs constructed with thermal bonds or other non-adhesive bonds can exhibit adequate bond strengths and desired absorbent properties, but slow production speeds and high bonding temperatures have been required to obtain the desired bond strengths. It has been difficult to construct the bonded webs at desired, high bonding speeds and high production rates. Additionally, it has been difficult to produce the thermal bonded webs with desired levels of flexibility and softness. As a result, there has been a continued need for improved bonded webs that can exhibit desired combinations of bond strength, flexibility, absorbency and softness, while also being capable of being constructed at high, production-bonding speeds.

### BRIEF DESCRIPTION OF THE INVENTION

The present invention provides a method as claimed in claim 1. Optionally, the web article can include a second fibrous layer laminated with a second facing surface of the at least one polymer film layer. In particular aspects, the first and/or second fibrous layers may include multiple plies of their corresponding fibrous materials.

The article provided by the invention can more efficiently provide a bonded web which can be produced at high speeds and exhibit desired levels of bond strength. Where the bonded web article is intended to provide an absorbent sheet article, the bonded web can exhibit sufficiently high bond strengths without excessively inhibiting the absorbent properties of the bonded web. Additionally, the bonded web articles of the invention can provide desired levels of flexibility and softness.

### BRIEF DESCRIPTION OF THE DRAWINGS

The invention will be better understood by reference to the following description of the invention taken in conjunction with the accompanying drawings, wherein:

FIG. 1 shows a schematic, side elevational view of a representative method and apparatus having a rotatable pattern roller and a counter-rotatable anvil roller.

FIG. 1A shows a schematic, perspective view of a representative method and apparatus having a rotatable pattern roller and a counter-rotatable anvil roller.

FIG. 2 shows a representative pattern of bonding pins on a pattern roller.

FIG. 2A shows individual bonding pins that extend a selected height from an outer surface of a pattern roller.

Fig. 2B shows a representative pattern of non-circular bonding pins on a pattern roller.

### DETAILED DESCRIPTION OF THE INVENTION

It should be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

As used herein, the term "nonwoven" refers to a fabric web that has a.. structure of individual fibers or filaments which are interlaid, but not in an identifiable repeating manner.

As used herein, the terms "spunbond" or "spunbonded fiber" refer to fibers which are formed by extruding filaments of molten thermoplastic material from a plurality of fine, usually circular, capillaries of a spinneret, and then rapidly reducing the diameter of the extruded filaments.

As used herein, the phrase "meltblown" fibers refers to fibers formed by extruding a molten thermoplastic material through a plurality of fine, usually circular, die capillaries as molten threads or filaments into a high velocity, usually heated, gas (*e.g.*, air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers.

"Coform" as used herein is intended to describe a blend of meltblown fibers and cellulose fibers that is formed by air forming a meltblown polymer material while simultaneously blowing air-suspended cellulose fibers into the stream of meltblown fibers. The meltblown fibers containing wood fibers are collected on a forming surface, such as provided by a foraminous belt. The forming surface may include a gas-pervious material, such as spunbonded fabric material, that has been placed onto the forming surface.

As used herein, the phrase "absorbent article" refers to devices which absorb and contain liquids. Particular devices can be placed against or near the skin to absorb and contain the various liquids discharged from the body. The term "disposable" is used herein to describe articles that are not intended to be laundered or otherwise restored or reused as an absorbent article after a single use. Disposable articles are typically intended for limited use, and are ordinarily discarded after the article has been soiled. Examples of such disposable absorbent articles include, but are not limited to: wipes, towels, health care related products including surgical drapes, gowns, covers and sterile wraps; personal care absorbent products such as feminine hygiene products (e.g., sanitary napkins, pantiliners, tampons, interlabial devices and the like), Infant diapers, children's training pants, adult incontinence products and the like; as well as absorbent wipes and covering mats.

As used herein, the phrase "wet laid" refers to the process form making non-woven webs prepared by suspending fibers in a liquid medium, such as water, applying the fibrous slurry to a forming wire or fabric, removing the liquid from the fibers to form a continuous fibrous web and drying the web. Wet-laid webs are well known In the art. Wet laid cellulosic webs are disclosed, for instance, In U.S. Pat. No. 3,879,257 to Gentile et al., U.S. Pat. No. 5,399,412, issued to S, J. Sudall and S. A. Engel on Mar. 21, 1995; and U.S. Pat. No. 5,672,248, issued to Wendt et al. on Sep. 30, 1997.

As used herein, the phrase "tissue" refers to tissue and towel base-sheets or products. "Tissue" is differentiated from other paper products in terms of its bulk. The bulk of the tissue and towel products of the present invention is calculated as the quotient of the caliper expressed in microns, divided by the basis weight, expressed in grams per square mater. The resulting bulk is expressed as cubic centimeters per gram. Writing papers, newsprint and other such papers have higher strength, stiffness and density (low bulk) in comparison to tissue which tends to have much higher caliper for a given basis weight. Tissue of the present invention may have a bulk of 2 $cm^3$/g or greater, more specifically 4 $cm^3$/g or greater, and still more specifically about 6 $cm^3$/g or greater.

The term "caliper" as used herein for tissue, refers to the thickness of a single tissue sheet, and may either be measured as the thickness of a single tissue sheet or as the thickness of a stack of ten tissue sheets and dividing the ten tissue sheet thickness by ten, where each sheet within the stack is placed with the same side up. Tissue caliper is expressed in $\mu$m. Caliper is measured in accordance with TAPPI test methods T402 "Standard Conditioning and Testing Atmosphere For Paper, Board, Pulp Handsheets and Related Products" and T411 om-89 "Thickness (caliper) of Paper, Paperboard, and Combined Board" optionally with Note 3 for stacked tissue sheets. The micrometer used for carrying out T411 om-89 is a Bulk Micrometer (TMI Model 49-72-00, Amityville, N.Y.) or equivalent having an anvil diameter of 4 1/16 inches (103.2 millimeters) and an anvil pressure of 220 grams/square inch (3.3 KPa).

The basis weight and bone dry basis weight of tissue specimens can be determined using a modified TAPPI T410 procedure or equivalent For example, as is basis weight samples are conditioned at 23°C $\pm$ 1°C and 50 $\pm$ 2% relative humidity for a minimum of 4 hours. After conditioning, a stack of 16, 3 inch X 3 inch (7.6 x 7.6 cm) samples are cut using a die press and associated die. This represents a tissue sheet sample area of 929 $cm^2$. Examples of suitable die presses are TMI DGD die press manufactured by Testing Machines, Inc. located at Islandia, NY, or a Swing Beam testing machine manufactured by USM Corporation, located at Wilmington, MA. Die size tolerances are +/- 0.008 inches (0.20 mm) in both directions. The specimen stack is then weighed to the nearest 0.001 gram on a tared analytical balance. The basis weight in g/$m^2$ is then calculated.

Disposable absorbent articles may include a plurality of components or layers. For example, the absorbent article can be a wipe, towel or the like, which includes two or more layers of selected materials. Other examples of the absorbent article can include a liquid pervious topsheet, a substantially liquid impervious backsheet connected to the topsheet, and an absorbent core positioned and held between the topsheet and the backsheet. The topsheet is operatively permeable to the liquids that are intended to be held or stored by the absorbent article, and the backsheet may be substantially impermeable or otherwise operatively impermeable to the intended liquids. The absorbent article may also include other components, such as liquid wicking layers, liquid distribution layers, barrier layers, and the like, as well as combinations thereof.

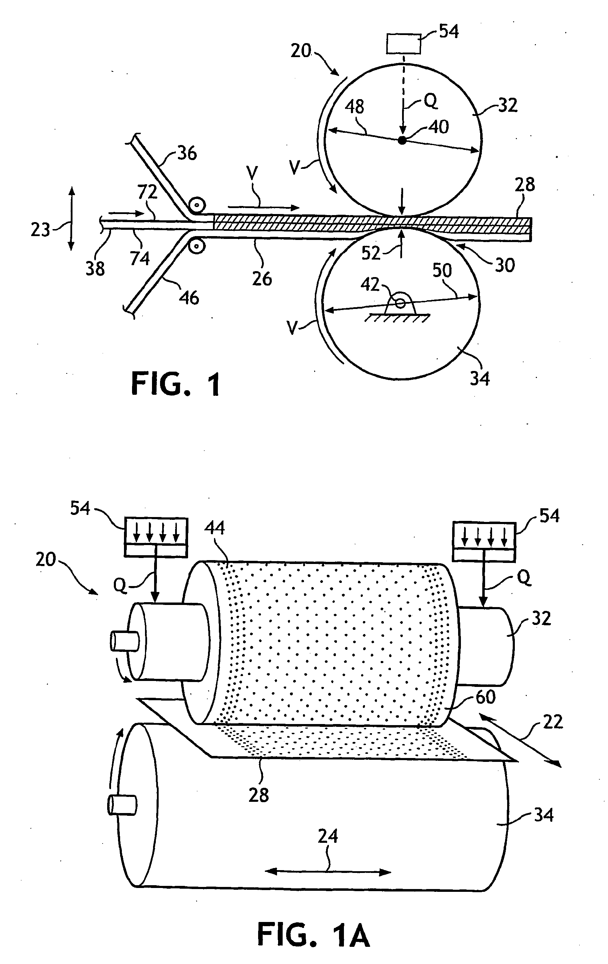

With reference to FIGs. 1 and 1A, the invention can provide a web article 28 having a first web of a selected, first material (e.g. fibrous layer 36) operatively bonded to at least a second web of a selected, second material (e.g. polymer film layer 38). Optionally, the bonded web may include one or more additional webs of material (e.g. layer 46). Any suitable materials may be employed. Such materials can, for example, include woven fabrics, nonwoven fabrics, layers of natural fibers, layers of synthetic fibers, spunbond fabrics, meltblown fabrics, carded-web fabrics, bonded-carded web fabrics, composite fabrics, polymer films, perforated polymer films, net materials, or the like, as well as combinations thereof. Examples of suitable nonwoven web materials can include web materials containing natural fibers, spunbond (SB) fabrics, spunbond-meltblown-spunbond (SMS) laminates, neck-bonded-laminates (NBL), Point UnBonded (PUB) fabrics, Vertical Filament Laminates (VFL), Stretch Bonded Laminates (SBL), or the like. The bonded web 28 may also include other materials. For example, such materials may include other absorbent natural fibers, superabsorbent polymer materials, absorbent synthetic fibers, or the like, as well as combinations thereof.

In particular arrangements, at least one of the first and second web materials employed to form the bonded web 28 can be a fabric or fibrous web material. The fibrous web material can include synthetic fibers or natural fibers, as well as combinations thereof. In desired arrangements, the fibrous web material can include cellulosic fibers, and the cellulosic fibers may include cotton fibers, woodpulp fibers or the like, as well as operative combinations thereof.

In other arrangements, at least one and optionally both of the employed web materials (e.g. layers 36, 38) can include polymeric materials. Desirably, the employed polymeric materials can operatively be heat processible and/or thermally bondable. For example, the selected polymeric web material can include polyester, polypropylene, polyethylene, nylon, polyolefins, such as copolymers of polypropylene and polyethylene, linear low-density polyethylene, aliphatic esters such as polylactic acid, other heat-processible or heat-bondable materials and the like, as well as combinations thereof.

Desirably, the invention can be configured to provide a web article having at least one nonwoven fabric or fibrous layer bonded to a polymer film material. As representatively shown in FIGs. 1 and 1A, the invention can be configured to provide a distinctive web article 28 which includes at least a first fibrous layer 36 laminated with a first side or facing surface 72 of at least one polymer film layer 38. Additionally, the first fibrous layer can include a first quantity of cellulosic fibers. The first fibrous layer 36 and the at least one polymer film layer have been configured to provide a target web 26. The target web has also been moved at a high web speed (V) through a nip region 30 between a rotating anvil roller 34 and a counter-rotating pattern roller 32 to provide an operative first bond between the at least one fibrous layer 36 and the at least one film layer 38. The anvil roller 34 and pattern roller 32 have been urged together to provide an operative nip force value; and the first bond between the at least one fibrous layer 36 and the at least one film layer 38 provides an operative, bond peel-strength value. In a particular aspect, the target web 26 has been moved at a web speed of at least a minimum of about 3.6 m/sec. In another aspect, the anvil roller and pattern roller have been urged together to provide a nip force, lineal pressure value, which is at least $0.05 \times 10^6$ N/m. In a further aspect, the lineal pressure value can be not more than $10 \times 10^6$ N/m. Still another aspect can provide a web article in which the bond between the at least one fibrous layer 36 and the at least one film layer 38 provides a bond peel-strength value of at least a minimum of 5 N/m. In a further aspect, the web article can comprise a second fibrous layer 46 laminated with a second facing surface 74 of the at least one polymer film layer 38.

By incorporating its various aspects and features, alone and in combination, the article of the invention can provide a distinctive, bonded web, which can exhibit desired levels of bond strength and can be more efficiently produced at high speeds. Where the bonded web article of the invention is intended to provide an absorbent sheet article, the bonded web can exhibit sufficiently high bond strengths without excessively inhibiting the absorbent properties of the bonded web. Additionally, the bonded web articles of the invention can provide desired levels of flexibility and softness. In desired arrangements, the web article of the invention can be incorporated into a selected absorbent article, and the absorbent article may be configured to be disposable.

In the various arrangements of the invention, the web materials can have any operative configuration. In a particular feature, at least one of the selected material webs can be a fabric or fibrous web having a basis weight which is at least a minimum of 6 g/m$^2$ (0.2 osy). The basis weight of the fibrous material can alternatively be at least 10 g/m$^2$ (about 0.3 osy), and can optionally be at least 12 g/m$^2$ (0.35 osy) to provide desired benefits. In other aspects, the fibrous basis weight can be up to a maximum of 350 g/m$^2$ (about 10 osy), or more. The basis weight can alternatively be up to 200 g/m$^2$ (about 5.9 osy), and can optionally be up to 150 g/m$^2$ (4.4 osy) to provide desired effectiveness.

It should be readily appreciated that each of the employed fibrous layer or layers may contain a single ply or stratum of a selected fibrous material. Optionally, each employed fibrous layer may contain a multiplicity of two or more plies or strata of the selected fibrous material. When the fibrous layer contains multiple plies of fibrous material, the plies may or may not be laminated or otherwise operatively attached to one another prior to bonding the fibrous layer to the polymer film.

In desired aspects, the employed fibrous layer (or layers) can include cellulosic material, such as cellulosic

EP 1 937 458 B1

tissue, but may or may not include synthetic fibers. In another aspect, the employed fibrous layer (or layers) can include a wet laid tissue. The employed cellulosic material may include any type of natural fiber, and the fiber furnish selection may be adjusted, depending upon the particular application for which the product or web article is intended. For example, if the sheet is intended to be used in paper towel type applications, the furnish may be a BCTMP (Bleached Chemical Thermo Mechanical Pulp, a softwood pulp, such as a NSWK (Northern Softwood Kraft) or combinations thereof. The tissue may also include recycled fibers. The cellulosic material can also have a selected basis weight. In a particular aspect, the basis weight can be at least a minimum of 10 g/m$^2$. The cellulosic basis weight can alternatively be at least 20 g/m$^2$. and can optionally be at least 30 g/m$^2$ to provide desired benefits. In another aspect, the cellulosic basis weight can be up to a maximum of 200 g/m$^2$, or more. The basis weight can alternatively be up to 150 g/m$^2$, and can optionally be up to 100 g/m$^2$ to provide further, desired benefits. It should be noted that the above basis weights refer to the basis weight of the complete cellulosic layer that is bonded to the polymer film and includes the combined basis weights of the Individual plies that are in the complete cellulosic layer.

When the cellulosic layer is configured to provide an absorbent tissue or towel article, the tissue or towel sheet can be made by any method known in the art, which can include an airlaying process, a wet-laying process, a coforming process or the like, as well as combinations thereof. For wet-laid cellulosic layers, the layer can also be made by any operative, wet-laid technique, and such techniques are well known in the art. Accordingly, the wet-laid cellulosic layer can, for example, be wet pressed, conventional through-air-dried, or uncreped-through-air-dried.

The uncreped-through-air-dried (UCTAD) cellulosic material can help provide greater wet resilience and absorbent capacity. Accordingly, the web article 28 can desirably Include at least one fibrous layer that includes the uncreped-through-air-dried cellulosic material. Suitable uncreped- through-air-dried materials are described in U.S. Patent 5,672,248; U.S. Patent 5,656,132; U.S. Patent 6,120,642; U.S. Patent 6,096,169; U.S. Patent 6,197,154; and U.S. Patent 6,143,135. The uncreped-through-air- dried materials can provide better bulk, greater wet resiliency and higher specific absorbent capacity than traditional wet pressed tissue products.

In particular configurations, the employed fibrous layer (36, 46) can include a through-air-dried tissue such as uncreped-through-air-dried tissue. In a desired arrangement, the absorbent cellulosic layer can include a through-air-dried tissue material having a basis weight within the range of 20 - 90 g/m$^2$. In another desired arrangement, the cellulosic layer can include a through-air-dried sheet material having high wet resiliency, such as the material described in U.S. Patent 6,808,790 B2 entitled WET-RESILIENT WEBS AND DISPOSABLE ARTICLES MADE THEREWITH by Chen et al., which issued October 26, 2004.

In other configurations, the employed fibrous layer (36,46) can include a pattern densified fibrous layer, such as a pattern densified through-air-dried tissue sheets. Examples of pattern densified fibrous structures are described In the following U.S. Patents: 4,514,345, issued on April 30, 1985 to Johnson et al.; 4,528,239, issued on July 9, 1985 to Trokhan; 5,098,522, Issued on March 24, 1992; 5,275,700, issued on January 4, 1994 to Trokhan; 5,328,565, issued on July 12, 994 to Rasch et al.; 5,334,289, issued on August 2, 1994 to Trokhan et al.; 5,431,786, issued on July 11, 1995 to Rasch et al.; 5,496,624, issued on March 5, 1996 to Steltjes, Jr. et al.; 5,624,790, issued on April 29, 1997 to Trokhan et al.; and, 5,628,876, issued on May 13, 1997 to Ayers et al. Such imprinted tissue sheets or print creped webs may have a network of densified regions that have been imprinted against a drum dryer by an imprinting fabric, and regions that are relatively less densified (e.g., "domes" in the tissue sheet) corresponding to deflection conduits in the imprinting fabric, wherein the tissue sheet superposed over the deflection conduits was deflected by an air pressure differential across the deflection conduit to form a lower-density pillow-like region or dome in the tissue sheet.

In still other configurations, the employed fibrous layer (36,46) may include a highly debonded cellulosic sheet and a latex binder. The sheet may be creped or uncreped. Such sheets generally have a smooth surface texture, high wet strength, high durability, low stiffness and a very clothlike feel and texture. While such sheets tends to have a lower specific absorbent capacity than the through air dried tissue sheets previously mentioned, the improved texture and feel may make them more suitable for some applications.

For example, In a particular arrangement, such cellulosic sheets can be prepared by 1) forming a wet laid tissue sheet, preferably debonded, 2) applying to one side of the web a latex comprising a binder material having a glass transition temperature of less than 40°C, 3) drying and creping said sheet such that the latex side is against the dryer, 4) applying to the second side of said sheet a latex binder material having a glass transition temperature of less than 40°c, 5) drying and creping said sheet such that the second side of said sheet is in contact with the dryer and 6) optionally curing said sheet to affect crosslinking of the binder. Examples of such tissue products and methods to make such products can be found in U.S. Patent No. 3,879,257 by Gentile, et. al entitled "ABSORBENT UNITARY LAMINATE-LIKE FIBROUS WEBS AND METHOD FOR PRODUCING THEM" assigned to Scott Paper Company and issued April 22, 1975. Such materials are at times referred to as Double Re-Creped or DRC sheets. A variety of binders are known in the art and may be used. Particularly suitable binders are styrene butadiene, polyvinylchloride and ethylene vinyl acetate binders such as EN-1165 and A-124 available from Air Products Corporation and ELITE PE Binder available from National Starch, Inc.

Various other processes are suitable for making such clothlike cellulosic structures. Acceptable products and process are described in commonly assigned U.S. Patent Application Nos. 10/192,781, filed July 10, 2002; 10/447,321, filed May 28, 2003; 10/326,915, filed December 20, 2002; 10/382,222, filed March 5, 2003; 10/319,415, filed December 13, 2002; and 10/749,475, filed December 31, 2003. The print creped and dual re-creped webs have a clothlike feel that can be characterized by the sheet's high tensile energy absorption (TEA) and low geometric mean tensile modulus (stiffness).

In particular arrangements the geometric mean tensile strength of the print creped web (without the fluid impervious layer), defined as the square root of the product of the machine-direction tensile strength and the cross-direction tensile strength can be 900 g per 3 inches (7.6 cm) of sample width (900 g/3"), or greater, such as from 900 g/3" to about 5000 g/3" in one embodiment to from 1000 g/3" to about 3000 g/3" in another embodiment. The geometric mean modulus of the print creped web without the fluid impervious layer, defined as the square root of the product of the machine direction tensile modulus and the cross direction tensile modulus is preferably less than about 11 kg such as less than 9 kg in one embodiment to about less than 7 kg in another embodiment. On the other hand the geometric mean tensile energy absorption defined as the square root of the product of the machine direction and cross direction tensile energy absorption is preferably greater than 15 $g\text{-}cm/cm^2$ such as greater than 17 $g\text{-}cm/cm^2$, such as from 17 $g\text{-}cm/cm^2$ to 40 $g\text{-}cm/cm^2$.

It should be readily appreciated that each of the employed cellulosic layer or layers may contain a single ply or stratum of a selected tissue or other cellulosic material. Optionally, each employed cellulosic layer may contain multiple plies or strata of the selected tissue or other cellulosic material.

The employed fibrous web layer can have a selected bulk, and the fibrous web bulk can desirably be provided in a non-woven, non-wet-laid web. The fibrous web bulk can be at least a minimum of 2 $cm^3/g$. The bulk can alternatively be at least 4 $cm^3/g$, and can optionally be at least 6 $cm^3/g$ to provide desired benefits. In other aspects, the bulk can be up to a maximum of 25 $cm^3/g$, or more. The bulk can alternatively be up to 16 $cm^3/g$, and can optionally be up to 12 $cm^3/g$ to provide desired effectiveness.

As previously described, at least one of the selected material webs employed to form the bonded web 28 can include any operative polymer film. The polymer film may, for example, be composed of polyethylene, polypropylene, polyester or the like, as well as combinations thereof. Additionally, the polymer film may be micro-embossed, and may be configured to be operatively liquid-impervious. In a particular feature, it has been found that the incorporation of a substantially liquid-impervious polymer film layer 38 can help increase the fiber absorbent capacity of the employed cellulose web layer or layers. In optional configurations, the polymer film can operatively permit a sufficient passage of air and moisture vapor through the thickness dimension of the film while blocking the passage of liquids. An example of a suitable polymer film material can include a breathable, microporous film. In a more particular example, the polymer film material can be a breathable film, which is white in color, dimple embossed, and contains: 47.78% calcium carbonate, 2.22% $TiO_2$, and 50% polyethylene.

The employed film layer 38 can have a selected basis weight of polymer film material. The film basis weight can be configured to provide desired barrier properties, such as an operative level of liquid-imperviousness. In a particular aspect, the film basis weight can be at least a minimum of 5 $g/m^2$. The film basis weight can alternatively be at least 8 $g/m^2$, and can optionally be at least 12 $g/m^2$ or at least 15 $g/m^2$ to provide desired benefits. In other aspects, the film basis weight can be up to 80 $g/m^2$. The film basis weight can alternatively be up to 70 $g/m^2$, and can optionally be up to 60 $g/m^2$ to provide desired benefits.

In another feature, the employed polymer film web can have a film thickness which is at least a minimum of 0.0005 inch (about 0.013 mm). The film thickness can alternatively be at least 0.0006 inch (0.015 mm), and can optionally be at least 0.0007 inch (0.018 mm) to provide desired benefits. In other aspects, the film thickness can be up to a maximum of 0.002 inches (0.051 mm), or more. The film thickness can alternatively be up to 0.0018 inch (0.046 mm), and can optionally be up to 0.0015 inch (0.038 mm) to provide desired effectiveness.

Lower weights and lower thicknesses of the polymer film can help to reduce cost as well as reduce any stiffness that may be imparted to the web article 28 by the polymer film. The thickness of the polymer film can desirably be balanced with the ability to avoid pinholes in the film sheet. Excessive pinholes can compromise and reduce the protective functionality of the article web.

A further feature can provide a web article 28 which has a significant amount of absorbent capacity for aqueous liquids. In a desired aspect, the fiber specific absorbent capacity of the web article can be at least about 4 grams of water per gram of the fibrous material in the fibrous layer or fibrous layers (36, 46) of the article. The fiber specific absorbent capacity can alternatively be at least 6 g/g fiber, and can optionally be at least 8 g/g fiber to provide improved benefits. In a further aspect, the saturated capacity can be up to a maximum of 20 g/g fiber, or more. The saturated capacity can alternatively be up to 18 g/g fiber, and can optionally be up to about 16 g/g fiber to provide desired benefits.

The saturated absorbent capacity is a measure of the total absorbent capacity of an absorbent body, absorbent garment, or other absorbent article or product. The saturated absorbent capacity of the fiber material in the article web 28 is a measure of the weight of water retained by a sample of the article web per weight of the fiber material in the fibrous layer or layers of the article web.

The tiber specific absorbent capacity, saturated absorbent capacity and fiber specific absorbent capacity can be determined as follows. Each sample should be conditioned at standard TAPPI conditions for a minimum of 4 hours prior to testing.

A sample specimen of the bonded web is cut from a ply by using a swing beam cutting machine (e.g. SB-20, USM Corporation, or equivalent) or a manual paper cutter (e.g. McMaster-Carr Supply Co. part # 3823A44, or equivalent). The sample measures 4 inches by 4 inches (10.16cm by 10.16 cm), and is weighed to the nearest 0.01 gram on a suitable balance. The "dry weight" of the sample specimen is recorded. A weighing dish is tared on the balance. The sample specimen is attached on one end with the minimum length needed to hang the specimen from a pinch type clamp or clip. A container is filled with a sufficient volume of water to insure that the test specimen and clamp can be fully submerged. The water temperature should be maintained at 23 ± 3°C. The specimen and clamp are then placed into the water absorbent side up using a tongs. A timer is immediately started. The specimen is allowed to soak in the water for 30 ± 5 seconds. The specimen is removed from the water by grasping the clamp with the tongs. The sample is then suspended in the air by hanging from the clamp for 30 ± 5 seconds, with the lower edge approximately parallel to the horizontal. The clamp is shaken lightly once, only to the extent needed to remove any drips hanging from the specimen.

Where the specimen is composed of a fibrous layer attached to only a single facing surface of the polymer film, the specimen is then released from the clamp, fibrous-side up, onto a blotter paper so as to remove any water from the polymer film-side. Care should be taken to avoid contacting the hydrophilic side of the sample with the blotter paper. The sample's polymer film-side should be completely contacting the blotter paper. The specimen is allowed to sit on the blotting paper for 3 ± 1 seconds. The specimen is then removed from the blotter, and weighed in a tared weighing dish.

Where the specimen includes a fibrous layer attached to each of the two outwardly facing surfaces of the polymer film, the actions pertaining to the blotting of the specimen are skipped and not conducted.

The weight of the wet sample "Wet Weight" is recorded to the nearest 0.01 gram. The absorbent capacity in g/cm$^2$, specific absorbent capacity (g-water / g-sample) or fiber absorbent capacity (g-water / g-fiber) can then be determined using the following equations:

Saturated Absorbent Capacity (g/cm$^2$) of specimen

$$= \frac{\text{Specimen Wet Weight (g)} - \text{Specimen Dry Weight (g)}}{\text{Sample area}}$$

Where: Sample area = 103.2 cm$^2$, for a 4" X 4" sample size.

Specific Absorbent Capacity (g/g sample) of specimen

$$= \frac{\text{Specimen Wet Weight (g)} - \text{Specimen Dry Weight (g)}}{\text{Specimen Dry Weight (g)}}$$

Fiber Specific Absorbent Capacity of fibrous layer (g/g fiber)

$$= \frac{\text{Fiber Wet Weight (g)} - \text{Fiber Dry Weight (g)}}{\text{Fiber Dry weight (g)}}$$

The Fiber Wet Weight and Fiber Dry Weight are determined by determining the weight of the polymer film in the specimen, and subtracting the polymer film weight from the Specimen Wet Weight and the Specimen Dry Weight, respectively.

When a non-uniform bonding pattern is applied to form the laminated web article 28, samples for determining the percent bonded area are taken such that the sample area is representative of the percent bonded area of the overall web article. If it is not possible to cut single samples representative of the bonding pattern in the web article, the fiber specific absorbent capacity is determined by randomly selecting 10, 4" X 4" (10.16 cm X 10.16 cm) specimens from the web article. The samples should be taken from different places in the machine and cross deckle positions of the web article. If the web article has been converted into sheets for a product such as a wiping implement or perforated such that individual sheets of product may be identified, such as in a spirally wound roll of perforated sheets as commonly used for paper towels, 4" X 4" (10.16 cm X 10.16 cm) samples are cut from 10 different sheets. The 4" X 4" (10.16 cm X 10.16 cm) samples are preferably taken from different positions in the machine and cross deckle positions of the sheets. The fiber specific absorbent capacity is determined for each of the 10 samples and the average value of the ten samples is used as the fiber specific capacity of the web article.

In still another aspect of the web article 28, the article can have a bonded area of not more than a maximum of 50% of the surface area of the web article. The bonded area can alternatively be not more than about 40%, and can optionally can be not more than 30%. In a desired arrangement, the bonded area can be up to 20%. Additionally, the article can have a bonded area of not less than a minimum of 3%. The bonded area can alternatively be not less than 5%, and can optionally be not less than 7%. The relatively low bonded area can help improve the liquid-handling properties of the article, and can help increase the flexibility of the article.

The bonded web 28 has been configured to have a desired bonding peel-strength value. In a particular aspect, the composite bonded web 28 can have a composite bond peel-strength value which is at least a minimum of 5 N/m. The bonding strength value can alternatively be at least 7 N/m, and can optionally be at least 8 N/m to provide desired benefits. In other aspects, the bonding strength value can be up to a maximum of 25 N/m, or more. The bonding strength value can alternatively be up to 20 N/m, and can optionally be up to 15 N/m to provide desired effectiveness.

The bond peel-strength values can be determined by following the procedures described in the ASTM D 1876-01 "*Standard Test Method for Peel Resistance ofAdhesives (T-Peel Test)*", with the following modifications. In the test specimens of the bonded webs, the machine-direction of the bond is arranged to extend along the width dimension of the test specimen, and the width dimension of the test specimen is perpendicular to the intended pulling direction of the tensile testing machine. The width of each test specimen measures 76.2 mm (3 inches), instead of 25 mm. A suitable tensile testing system is a MTS ALLIANCE RT/1 machine, which is available from MTS Systems Corporation, a business having offices located in Eden Prairie, Minnesota, U.S.A. A substantially equivalent tensile tester may alternatively be employed. The gage length (the separation distance between the two grips of the tensile tester) is set to $50 \pm 1$ mm. The test specimen is gripped such that the manufacturing, machine-direction of the bonded seam region is substantially perpendicular to the pulling direction of the tensile testing machine. The tensile tester applies the testing load at a constant head speed of $508 \pm 10$ mm/min until the bond (seam) fails or detaches. To determine the bond strength of a particular bonded web, 6 test specimens of the bonded web are prepared and tested to determine the peak force value obtained from each test specimen. The six peak-force values are arithmetically averaged to determine the bond strength of the particular bonded web. The bond strength value and the standard deviation (Stdv) of the corresponding peak-force values are outputted in the units of kilogram (kg), which corresponds to the total force needed to beak the 3-inch (76.2 mm) wide bond. It should be noted that for the 3-inch (76.2 mm) wide specimen, a bonding strength value of 1 kg corresponds to a bonding strength value of 131 N/m.

Additional information and details regarding the peel test procedure are set forth in ASTM D 1876-01 "*Standard Test Method for Peel Resistance of Adhesives (T-Peel Test)*". Additional information regarding the MTS tensile tester can be obtained from MTS Systems Corporation, Eden Prairie, Minnesota, U.S.A.

With reference to FIGs. 1 and 1A, the web article 28 can further include at least a second fibrous layer 46 laminated with a second facing surface or side of the at least one polymer film layer 38. Accordingly, the target web 26 has been configured to further include the second fibrous layer 46 positioned on the second facing surface of the at least one polymer film layer. Additionally, this target web has been moved at the web speed through the nip region 30 between a rotating anvil roller 34 and a counter-rotating pattern roller 32 to provide an operative second bond between the second fibrous layer 46 and the at least one polymer film layer.

It should be readily apparent that the materials, characteristics and parameters of the second fibrous layer 46 can be the same as or different than the materials, characteristics and parameters that are disclosed for the first fibrous layer 36. For example, the second fibrous layer 46 can also include cellulosic material, and the characteristics and parameters of the cellulosic material in the second fibrous layer 46 can be the same as or different than the characteristics and parameters of the cellulosic material in the first fibrous layer 36. Similarly as another example, the second bond between the second fibrous layer and the employed polymer film layer can have bonding characteristics and parameters that are the same as or different than the bonding characteristics and parameters of the first bond between the first fibrous layer 36 and the employed polymer film 38.

As disclosed herein, the target web 26 can be moved at a relatively high web speed during the production of the article web 28. In other aspects, the anvil roller and pattern roller can be urged together to provide a distinctive nip force value. A further aspect can provide a web article in which the bond between the employed fibrous layer (36, 46) and the employed film layer (38) has a sufficiently high bond peel-strength value. In a particular feature, the bond peel-strength value can be provided even when the percentage of bonded area in the article web is relatively low.

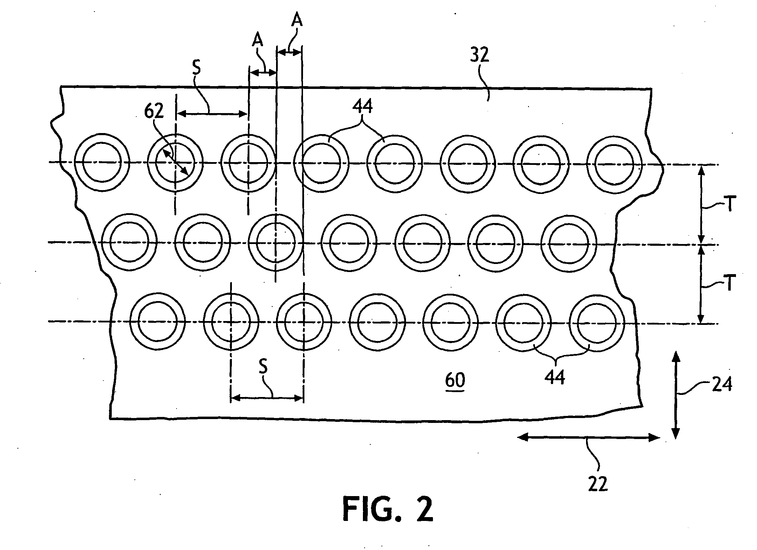

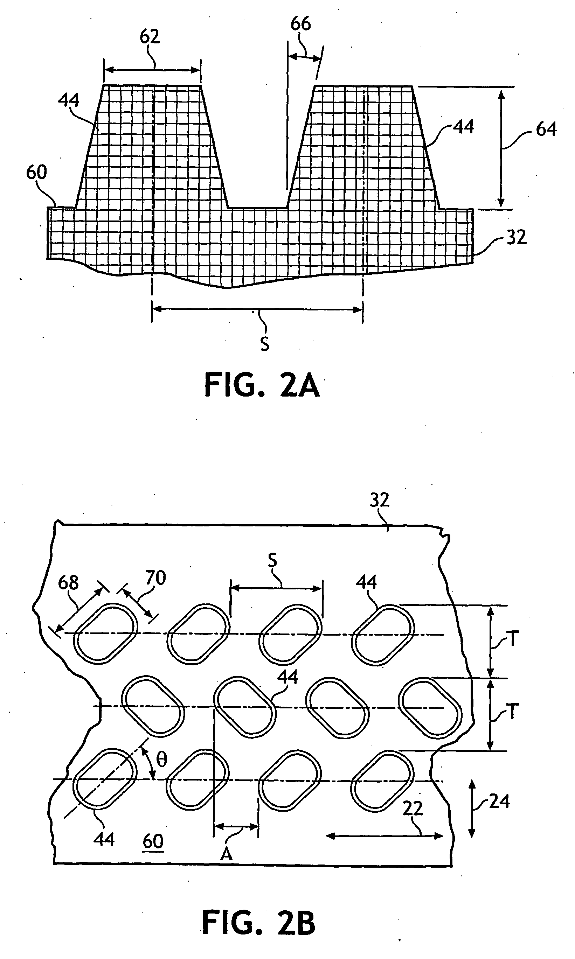

With reference to FIGs. 1 through 2B, a process and apparatus 20 for making an article 28 of the invention can have a lengthwise, machine-direction 22 which extends longitudinally, a lateral cross-direction 24 which extends transversely, and an appointed z-direction 23. For the purposes of the present disclosure, the machine-direction 22 is the direction along which a particular component or material is transported length-wise along and through a particular, local position of the apparatus and method. The cross-direction 24 lies generally parallel to the local horizontal, and is aligned perpendicular to the local machine-direction 22. The z-direction is aligned substantially perpendicular to both the machine-direction 22 and the cross-direction 24, and extends generally along a depth-wise, thickness dimension of the appointed material targeted for work.

A suitable bonding method and apparatus 20 for producing the bonded web 28 can include a delivering of a target web 26 having one or more selected bonding materials through a nip region 30 between at least one cooperating pair of rotatable bonding rollers, thereby forming and producing the bonded web 28. In a particular aspect, a pattern roller 32 can be configured to provide a distinctively high, pattern surface speed. In another aspect, the pair of bonding rollers can be configured to provide a distinctively low, bonding pressure value. In further aspects, an anvil roller 34 can be provided with an anvil surface speed, and the anvil surface speed can be configured to be substantially equal to the pattern surface speed.

The method and apparatus 20 can provide a low cost and low maintenance, rotary pressure bonding (RPB) technology. The method and apparatus can more efficiently produce bonds having a desired, sufficiently high strength value, In particular features, the sufficient level of bonding can be produced while operating at relatively low pressure values and at very high speeds. In other aspects, the method and apparatus can produce distinctively Interconnected bonds having high, attachment strength values. The desired bonding can also be accomplished while operating at ordinary room temperatures. Additionally, the method and apparatus can produce adequate bonding between work materials that are ordinarily considered to be incompatible, particularly when employing conventional thermal or ultrasonic bonding techniques. It should be readily appreciated that the method and apparatus can be employed in any suitable manufacturing system that Includes a hlgh-speed bonding of selected web materials. For example, the method and apparatus can be employed in the construction of reusable articles, disposable articles or disposable absorbent articles or the like, as desired.

Details regarding a suitable bonding method and apparatus, and suitable web materials are described in U.S. Patent Publication Number 2006-0266473 entitled BONDING BY INDUCED HIGH-RATE OF SHEAR DEFORMATION by N. Senapati et al, which was filed May 26, 2005.

The target web 26 employed with the method and apparatus 20 can include one or more layer regions provided by selected materials. For example, the target web can include a first web of a selected, first material 36 and at least a second web of a selected, second material 38. The first and second materials can be different or substantially the same. Optionally, the target web may include one or more additional webs of material. Any suitable web of material may be employed, Such webs can, for example, include woven fabrics (fibrous webs), nonwoven fabrics, spunbond fabrics, meltblown fabrics, carded-web fabrics, bonded-carded web fabrics, composite fabrics, polymer films, perforated polymer film webs, net materials, or the like, as well as combinations thereof. Examples of suitable nonwoven webs can include webs containing cellulosic fibers, spunbond (SB) fabrics, meltblown fabrics, coform fabrics spunbond-meltblown-spunbond (SMS) laminates, neck-bonded-laminates (NBL), Point UnBonded (PUB) fabrics, Vertical Filament Laminates (VFL), Stretch Bonded Laminates (SBL), or the like. The cellulosic fibers can include cotton fibers, woodpulp fibers or the like, as well as operative combinations thereof. The target web 26 may also include other materials. For example, such materials may include other absorbent natural fibers, superabsorbent polymer materials, absorbent synthetic fibers, or the like, as well as combinations thereof.

In desired arrangements, at least one of the first and second webs employed to form the bonded web 28 can be a fabric or fibrous web. Accordingly, the invention can be configured to provide a nonwoven fibrous layer or fabric bonded to a film material. In another feature, at least one and desirably both of the employed webs can include polymeric materials. Desirably, the employed polymeric materials can operatively be heat processible and thermally bondable. For example, the selected polymeric web material can include polyester, polypropylene, polyethylene, nylon, or other heat-bondable materials, polyolefins, such as copolymers of polypropylene and polyethylene, linear low-density polyethylene, aliphatic esters such as polylactic acid, and the like, as well as combinations thereof.

The method and apparatus 20 can include at least one cooperating pair of counter-rotatable or counter-rotating bonding rollers, and the bonding rollers can include at least one rotatable pattern roller 32 and at least one rotatable anvil roller 34. The pattern roller 32 has an operative axis of rotation 40, and can be provided with selected plurality of pattern bonding elements 44, which may be arrayed or otherwise arranged in any operative distribution. Such distributions of bonding elements are conventional and well known. The individual pattern bonding elements can, for example, be pin elements, and the pin elements can have any operative, size, shape and/or cross-section.

The method and apparatus can provide a distinctively high deformation rate or strain rate (length per length, per unit time) during the high-speed compression of the target web. The compressive mechanical deformation can induce an internal heating of the target web 26, and cause a temperature increase within the deformed materials of the target web. At the same time, thermal conduction can transfer heat away from the deformed target web materials. As a result, the net temperature rise within the deformed target web will be determined by the difference between the internal heating due to the web deformation, and the heat loss due to thermal conduction. At conventional previously employed, low bonding speeds, a large amount of heat can transfer away from the deformed material into the cooler parts of the target web and into the bonder rollers during the deformation period of the bonding process. As a result, the strain-induced heating can be excessively limited, and the conventional bonding systems have required auxiliary heating to develop a sufficient bond strength.

The method and apparatus can provide a distinctively high rate of mechanical deformation or mechanical strain, which produces an increased rate of internal heating that is significantly higher than the rate of heat loss due to thermal conduction. At high bonding speeds, mechanical deformation induced internal heating is so rapid that only a small amount of heat is lost due to thermal conduction. In a particular feature, the internal heating process can be nearly adiabatic. When the nip force is sufficiently high and sufficiently large local deformations are generated within the areas to be bonded, an auxiliary, pre-heating of the target web or bonding rollers is not needed during the bonding operation to obtain an adequate bond strength. It has been found that for a given nip force value and set of bonder equipment, the deformation strain rate can increase approximately linearly with respect to the bonding speed. Additionally, the deformation rate can increase approximately linearly with respect to a decrease of the diameter the pattern roller 32 or anvil roller 34. In a particular feature of the apparatus and method, the deformation strain rate (length per length, per unit time) during the bonding compression of the target web materials can, for example, be within the range of $1*10^3$ to $1*10^4$ sec$^{-1}$.

The thermal properties of the materials employed to construct the bonder rollers can also affect the amount of temperature increase produced within webs being deformed by the pressure bonding system. The larger the thermal conductivity of the bonder rollers, the smaller the temperature increase within the deformed materials of the target web for a given set of bonding conditions. Materials with a small thermal conductivity are desired, if the bonder is to be conducted without a significant amount of auxiliary heating. For example, the lower thermal conductivity of bonding rollers having peripheral bonding surfaces composed of steel can be lower than the thermal conductivity of bonding rollers having peripheral surfaces composed of copper. The bonding rollers with peripheral bonding surfaces composed of materials having the relatively smaller thermal conductivity coefficients can help improve the performance of the method and apparatus. The lower thermal conductivity can help reduce the need for auxiliary heating, and the bonding rollers can more efficiently raise the web materials towards their melting points to generate desired bonding strengths.

The pattern roller can also be provided with a selected pattern-roll diameter 48 and can be constructed from any operative material. In particular aspects, the pattern-roll diameter can be at least a minimum of 3 inch (76 mm). The pattern-roll diameter can alternatively be at least 4 inch (102 mm), and can optionally be at least 6 inch (152 mm) to provide desired benefits. In other aspects, the pattern-roll diameter can be up to a maximum of 24 inch (610 mm), or more. The pattern-roll diameter can alternatively be up to 18 inch (457 mm), and can optionally be up to 12 inch (305 mm) to provide desired effectiveness. In desired configurations, for example, the pattern-roll diameter can be 6.1 inch, 6.5 inch, 6.9 inch, or 12 inch (156 mm, 166 mm, 176 mm, or 304 mm; respectively). Where the pattern-roll diameter is within the desired values, the method and apparatus can be more compact, and can reliably and efficiently provide desired bonding strengths.

With regard to the diameters of the employed pattern roller 32 and anvil roller 34, it can be observed that for a given effective (or equivalent) nip force value, the bond strength of webs bonded with larger diameter bonding rollers, e.g. with 12 inch (304 mm) pattern and anvil rollers, can be lower than the bond strength of webs bonded with a smaller diameter bonding rollers, e.g. with a 7 inch (176 mm) pattern roller and a 6.5 inch (165 mm) anvil roller. Without intending to be bound by any particular theory, it is believed that an important factor is the deformation (strain) rate of the webs passing through the nip region 30 between the rotating pattern and anvil rollers. At a given surface speed, the deformation rate increases when the diameters of the pattern and anvil rollers decrease. Therefore, for a given nip force value and bonding speed, the relatively smaller diameter rollers can provide a relatively larger deformation rate and a relatively larger deformation strain. The larger plastic deformation can lead to a larger amount of plastic flow, and the larger amount of plastic flow can provide a greater bond strength. Other factors may contribute to this result. For example, some web materials can be more rate-sensitive than other materials. Additionally, the plastic deformation of the web material can be non-linear and dependent on the deformation history of the material. Since a significant amount of heat is generated during the dynamic plastic deformation, the thermal properties of the web material may also have a significant role.

As representatively shown in FIG. 1A, the pattern roller 32 can be configured to include a shaft member 56 and plurality of two or more pattern sleeves 58. The illustrated example includes a pair of substantially identical pattern sleeves. The outer diameter of the pattern sleeves provides the diameter of the pattern roller, and the bonding elements 44 can be operatively distributed on the outer peripheral surface 60 of the pattern roller 32 (e.g. on the outer peripheral surface of the pattern sleeves 58). Alternatively, the pattern roller 32 can have the form of a generally unitary cylinder with the selected pattern of bonding elements distributed on and along the outer surface of the cylinder. For example, two substantially identical patterns of bonding elements can be fabricated on the outer surface of the cylinder and spaced apart by a desired distance along the axial direction of the cylinder.

The pattern roller can have an array of bonding elements distributed on an outer surface of the pattern roller. The individual bonding elements may have any operative configuration, and any operative array may be employed. The array of bonding elements may be distributed in a pattern that is regular, irregular, linear, curvilinear, nonlinear, or the like, as well as combinations thereof. Techniques for constructing the individual bonding elements and the distributed, pattern arrays are conventional and well known in the art.

With reference to FiGs. 2 and 2A, the employed bonding pins or other bonding elements 44 can be configured with a generally tapered cylindrical shape having a pin diameter 62, a pin height 64 and a pin taper angle 66.

In particular aspects, the pin diameter 62 can be at least a minimum of 0.015 inch (0.38 mm). The pin diameter can alternatively be at least 0.025 inch (0.64 mm), and can optionally be at least 0.03 inch (0.76 mm) to provide desired benefits. In other aspects, the pin diameter can be up to a maximum of 0.25 inch (6.4 mm), or more. The pin diameter can alternatively be up to 0.15 inch (3.8 mm), and can optionally be up to 0.08 inch ( 2.03 mm) to provide desired effectiveness.

In further aspects, the pin height 64 can be at least a minimum of 0.004 inch (0.1 mm). The pin height can alternatively be at least 0.015 inch (0.38 mm), and can optionally be at least 0.025 inch (0.64 mm) to provide desired benefits. In still other aspects, the pin height can be up to a maximum of 0.25 inch (6.4 mm), or more. The pin height can alternatively be up to 0.1 inch (2.54 mm), and can optionally be up to 0.05 inch (1.27 mm) to provide desired effectiveness.

The pin taper angle 66 can be as low as 0°. The pin taper angle can alternatively be at least 10°, and can optionally be at least 15° to provide desired benefits. In other aspects, the pin taper angle 66 can be up to a maximum of 60°, or more. The pin taper angle can alternatively be up to 30°, and can optionally be up to 22.5° to provide desired effectiveness.

The distribution pattern of the bonding elements 44 can have a machine-direction (MD) pin spacing distance S along the circumferential direction of the pattern roller, a cross-direction (CD) pin spacing distance T along the axial direction of the pattern roller, and a MD pin offset spacing distance A along the circumferential direction of the pattern roller. When the pins are substantially circular, for example, the MD pin spacing distance S can be as low as a minimum distance determined by the formula:

$$S = d + (2*H* \tangent (\alpha))$$

where:

d = pin diameter (62);
H = pin height (64);
$\alpha$ = pin taper angle (66).

In other aspects, the MD pin spacing distance can be up to a maximum distances determined by the formula:

$$S = (5*d) + (10*H* \tangent(\alpha)).$$

Alternatively, the MD pin spacing distance can be up to a maximum distance determined by the formula:

$$S = (2*d) + (4*H *\tangent(\alpha)).$$

Optionally, the MD pin spacing distance can be up to a maximum distance determined by the formula:

$$S = (1.5*d) + (2*H* \tangent(\alpha)).$$

In further aspects, the CD pin spacing distance T can be as low as a minimum distance determined by the formula:

$$T = (0.5 *d) + (2*H* \tangent (\alpha))$$

where:

H = pin height (64);
$\alpha$ = pin taper angle (66).

Alternatively, the CD pin spacing distance T can be as low as a minimum distance determined by the formula:

$$T = (0.75 * d) + (2 * H * \text{tangent}(\alpha))$$

In still other aspects, the CD pin spacing distance can be up to a maximum distance determined by the formula:

$$T = (5 * d) + (10 * H * \text{tangent}(\alpha)).$$

Alternatively, the CD pin spacing distance can be up to a maximum distance determined by the formula:

$$T = (2 * d) + (4 * H * \text{tangent}(\alpha))$$

Optionally, the CD pin spacing distance can be up to a maximum distance determined by the formula:

$$T = (1.25 * d) + (2 * H * \text{tangent}(\alpha))$$

The MD pin offset distance A can be as low as zero. The MD pin offset distance can alternatively be at least a minimum distance determined by the calculation:

A = 0.5*d; where d = pin diameter (62); and

can optionally be at least the distance determined by the calculation:

$$A = 0.7 * d,$$

to provide desired benefits.

In other aspects, the MD pin offset distance A can be as large as the MD pin spacing distance S. The MD pin offset distance can alternatively be up to a maximum of one pin diameter, d, and can optionally be up to 0.8*d, to provide desired effectiveness.

The pattern of bonding elements can be configured to have any operative distribution. The pattern may be intermittent, arranged in two or more discrete segments, or substantially continuous along the circumferential machine-direction 22 of the pattern roller. Additionally, the pattern may be intermittent, arranged in two or more discrete segments, or substantially continuous along the axial cross-direction 24 of the pattern roller.

The pattern of bonding elements may also include any operative number of pin lines that extend circumferentially along the machine-direction 22. In particular aspects, the number of pin lines can be at least a minimum of 1. The number of pin lines can alternatively be at least 2, and can optionally be at least 3 to provide desired bonding. In other aspects, the number of pin lines can be selected and arranged to provide a desired distribution and/or pattern of bonds across the surface area of the bonded web 38. In particular arrangements, the number of pin lines can be 50, or more. The number of pin lines can alternatively be 70, or more; and can optionally be 100, or more, to provide desired effectiveness. As representatively shown in FIGs. 2 and 2B, the pattern of bonding elements can be arranged to provide an operative multiplicity of circumferentially-extending lines of bonding elements 44 (e.g. lines of pins).

The peripheral bonding surface of an individual bonding element can have a selected bonding surface area. In particular aspects, the bonding surface area can be at least a minimum of 0.5 mm$^2$. The bonding surface area can alternatively be at least 1 mm$^2$, and can optionally be at least 2 mm$^2$ to provide desired benefits. In other aspects, the bonding surface area can be up to 100 mm$^2$, or more. The bonding surface area can alternatively be up to 50 mm$^2$, and can optionally be up to 10 mm$^2$ to provide desired effectiveness.

Another aspect of the method and apparatus can include distinctive pin area fraction B which can be determined by the following formula:

$$B = G \div (S * T)$$

where:

G = bonding surface area of an individual bonding element (e.g. bonding surface area of an individual pin);

S = MD pin spacing distance along the circumferential direction of the pattern roller;

T = CD pin spacing distance along the axial direction of the pattern roller: For circular pins; G = (π *d²) ÷ 4. Accordingly, for circular pins:

$$B = (\pi * d^2) \div (4 * S * T)$$

where:

π = 3.14;

d = pin diameter (62).

In particular aspects, the pin area fraction can be at least a minimum of 0.04. The pin area fraction can alternatively be at least 0.15, and can optionally be at least 0.25 to provide desired benefits. In other aspects, the pin area fraction can be up to a maximum of 0.75, or more. The pin area fraction can alternatively be up to 0.5, and can optionally be up to 0.35 to provide improved effectiveness.

Alternative arrangements of the method and apparatus can include non-circular bonding elements, as representatively shown in FIG. 2B. For example, the bonding elements can be provided by pins having a generally oval-shape, with a relatively longer axis 68 and a relatively shorter axis 70. The longer axis 68 can be oriented at any operative slant angle θ relative to the machine-direction 22. The slant angle can be within the range of ± 80° relative to the machine-direction, and can desirably be within the range of ± 45° relative to the machine-direction to provide desired performance.

Where the pin sizes, pin shapes, pin spacings, pattern distributions and other parameters of the bonding elements are within the desired values, the method and apparatus can more reliably and more efficiently provide desired bonding strengths.

The anvil roller 34 has an operative axis of rotation 42, and can be constructed from any operative material. Additionally, the anvil roller can be provided with a selected anvil-roll diameter 50. In particular aspects, the anvil-roll diameter can be at least a minimum of 3 inch (76 mm), and can alternatively be at least about 5 inch (127 mm) to provide desired benefits. In other aspects, the anvil-roll diameter 50 can be up to a maximum of 24 inch (610 mm), or more, and can alternatively be up to about 12 inch (305 mm) to provide desired effectiveness. For example, desired arrangements can include an anvil-roll diameter 50 of 6.06 inch, 6.14 inch, 6.5 inch or 12 inch (154 mm, 156 mm, 165 mm or 304 mm, respectively).

Where the anvil-roll diameter is within the desired values, the method and apparatus can be more compact, and can reliably and efficiently provide desired bonding strengths.

The anvil roller can have an outer peripheral anvil surface which is substantially smooth, and substantially free of discrete bonding elements. Optionally, the anvil roller surface may include an operative array of anvil bonding elements. The anvil bonding elements can be configured to cooperate with the array of pattern elements 44. For example, the anvil bonding elements may have exactly the same or similar pattern of pin elements with less pin height. Alternatively, the anvil bonding elements may have exactly the same or similar pattern of pin elements, with less pin height and larger pin diameter. Optionally, the anvil bonding elements may have a pattern of aperturing elements to aperture holes on the appointed target webs.

An operative drive technique or system may be employed to counter-rotate, and cooperatively phase the bonding rollers. Such techniques and systems are conventional and well known, and are available from commercial vendors. In desired arrangements, the drive systems can operatively synchronize the rotations of the bonding rollers.

The target web and its associated materials are operatively transported or otherwise delivered through the nip region 30 between the cooperating pattern roller 32 and anvil roller 34 to form the desired bonded web. Any transport or delivery system or technique may be employed. Conventional systems and mechanisms, such as roller systems, belts and conveyors, are well known and available from commercial vendors.

The nip region 30 between the bonding rollers can be a variable nip gap distance or a substantially fixed, nip gap distance. Desirably, the method and apparatus can be configured to provide a variable nip gap. The variable nip can self-adjust, depending on the thickness and mechanical properties of the target web 26, the pin pattern parameters, and the total force applied in the nip region.

In a desired aspect, the nip region can be configured to provide a desired, operative nip gap distance 52, and the nip gap distance can be as low as zero millimeters. In other aspects, the nip gap distance can be up to a maximum of 5 mm, or more. The nip gap can alternatively be up to 0.5 mm or 1 mm, and can optionally be up to 0.05 mm or 0.1 mm to provide desired effectiveness.

The pattern roller 32 can be provided with a selected, pattern surface speed, and in a particular aspect, the pattern surface speed can be at least a minimum of 700 ft/min (3.6 m/sec). The pattern surface speed can alternatively be at least 800 ft/min (4.1 m/sec), and can optionally be at least 900 ft/min (4.6 m/sec) to provide desired benefits. In other aspects, the pattern surface speed can be up to a maximum of 3000 ft/min (15.2 m/sec), or more. The pattern surface speed can alternatively be up to 2000 ft/min (10.2 m/sec), and can optionally be up to 1600 ft/min (8.1 m/sec) to provide improved effectiveness.

If the pattern surface speed is outside the desired values, the generated bond strength can be excessively low or exhibit excessive variation. Additionally, excessive breakage of the target web may occur.

In a desired arrangement, the speed of the target web through the nip region 30 can be configured to be substantially equal to the pattern surface speed. Additionally, the anvil roller can be provided with a selected, anvil surface speed, and the pattern surface speed can be configured to be substantially equal to the anvil surface speed. It should be readily appreciated that some differential between the pattern surface speed and the anvil surface speed may occur. In a particular feature, the pattern surface speed may differ from the anvil surface speed by a speed differential that is not more than 1.5%, as determined with respect to the anvil surface speed. If the surface speed differential is outside the desired values, the bonds may be excessively weak, or the target web may exhibit excessive tearing, breaking or other damage.

A forcing mechanism 54 can be employed to provide a desired bonding pressure value in the nip region 30 between the pattern roller 32 and the anvil roller 34. Any operative forcing mechanism may be employed, and suitable forcing mechanisms are well known in the art and available from commercial vendors. Such forcing mechanisms can, for example, include hydraulic cylinders, pneumatic cylinders, weights, springs and the like, as well as combinations thereof. The forcing mechanism 54 can be configured to provide a variable force or a substantially constant force or pressure value in the nip region 30. Desirably, the method and apparatus can be configured to provide a substantially constant nip force or nip pressure value in the nip region. Suitable forcing mechanisms are conventional and available from commercial vendors.

The bottom roller can be the anvil roller 34, and can be fixed onto a base frame of the method and apparatus, and the top roller can be the pattern roller 32 which is configured to ride on the anvil roller. As representatively shown, the forcing mechanism 54 can include a pair of pneumatic air pressure cylinders which are configured at the ends of the shaft of the pattern roller 32 to symmetrically exert a desired, total force which urges the pattern roller towards the anvil roller. Alternatively, the spatial positions of the pattern roller and anvil roller can be exchanged or otherwise rearranged, and the anvil roller can be fixed to the base and positioned above the pattern roller. Optionally, the pattern roller may be offset along the machine-direction or at any angle with respect to the machine-direction (horizontal-direction) towards either side of the anvil roller. The air pressure cylinders can then be installed to operatively exert a loading force that pushes or otherwise urges the pattern roller against the anvil roller.

In the nip region 30 between the pattern roller 32 and the anvil roller 34, the invention can be configured to provide a distinctive lineal-pressure value, F, which has the units of force per lineal distance, and may also be referred to as the nip force value. The lineal-pressure value can be determined by the formula:

$$F = Q_T/L;$$

where:

$Q_T$ = the total force which is exerted by the forcing mechanism 54 to urge the pattern roller 32 towards contact with the anvil roller 34; and

L = an average length of contact between the peripheral bonding surfaces of the bonding elements and the surface of the anvil roller 34 if the pattern roller contacted the anvil roller, as measured along the axial cross-direction of the anvil roller.

The average length of contact, L, can be determined by the following formula:

$$L = n*G/S$$

where:

n = the number of pin lines;
G = bonding surface area of an individual bonding element (e.g. bonding surface area of an individual bonding pin);
S = the MD pin spacing distance.

For circular pins; G = ($\pi$ *d$^2$) ÷ 4. Accordingly, for the circular pins:

$$L = (n* \pi *d^2)/(4*S)$$

where:

    d = the pin diameter (62);
    $\pi$ = 3.14.

In particular aspects, the lineal-pressure value can be at least a minimum of 0.05 *10$^6$ N/m. The lineal-pressure value can alternatively be at least 0.5 *10$^6$ N/m, and can optionally be at least 1 *10$^6$ N/m to provide desired benefits. In other aspects, the lineal-pressure value can be up to a maximum of 10 *10$^6$ N/m, or more. The lineal-pressure value can alternatively be up to 5 *10$^6$ N/m, and can optionally be up to 3 *10$^6$ N/m to provide desired effectiveness.

If the bonding, lineal-pressure is outside the desired values, the generated bonds may be excessively weak, or the target web may become overbonded. Undesired holes may be punched through the target web, and excessive material can build up on the bonding pins.

The method and apparatus can be configured to provide effective bonding strengths even when operated at ambient room temperatures. Typical ambient room temperatures can, for example, be within the range of 18-32 °C. As a result, the invention can operatively provide desired bonding strengths without incorporating or otherwise subjecting the bonding operation to auxiliary heating. It has been found that the technique of the method and apparatus can distinctively employ a very rapid, high speed application of the selected bonding nip force (e.g. lineal-pressure value) in a manner that can efficiently and effectively bond webs having similar or dissimilar compositions.

The method and apparatus can be configured to provide effective bonding strengths even when operated at ambient room temperatures. Typical ambient room temperatures can, for example, be with the range of 18-32 °C. As a result, the invention can operatively provide desired bonding strengths without incorporating or otherwise subjecting the bonding operation to a separately provided, non-ambient, auxiliary heating. In a particular aspect, the bonding process can be substantially free of auxiliary heating conducted or applied at an auxiliary-heat temperature that is lower than the melting points of the web materials in the target web, and greater than 45 °C or 55 °C. The bonding can alternatively be substantially free of auxiliary heating applied at a temperature that is greater than 65 °C or 75 °C, and can optionally be substantially free of auxiliary heating applied at a temperature that is greater than 85 °C or 95 °C. It has been found that the technique of the method and apparatus can distinctively employ a very rapid, high speed application of the selected bonding nip force (e.g. lineal-pressure value) in a manner that can efficiently and effectively fuse, interlock or otherwise bond webs of materials having similar or dissimilar compositions.

Where the web article 28 includes a polymer film layer bonded to a fibrous layer that includes cellulosic fibers, the bonding method and apparatus may optionally include a selected level of auxiliary heating. The auxiliary heating may be applied by employing heated rollers, non-contact heating, radiant heating, hot air or the like, as well as combinations thereof.

The auxiliary heating can operatively subject the target web 26 to an auxiliary heating temperature during the high-speed pressure bonding of the target web. In particular aspects, the auxiliary heating temperature can be at least a minimum of 35 °C. The auxiliary heating temperature can alternatively be at least 40 °C, and can optionally be at least 45 °C to provide desired benefits. In other aspects, the auxiliary heating temperature can be up to a maximum of 250 °C, or more. The auxiliary heating temperature can alternatively be up to about 150 °C, and can optionally be up to 100 °C to provide desired effectiveness.

The following examples describe particular configurations of the invention, and are presented to provide a more detailed understanding of the invention. The examples are not intended to limit the scope of the present invention in any way. From a complete consideration of the entire disclosure, other arrangements within the scope of the claims will be readily apparent to one skilled in the art.

<u>Examples</u>

Laminates composed of an UCTAD (uncreped-through-air-dried) paper towel web and polymer film web were made by (a) an adhesive bonding process, (b) a thermal embossing/bonding process and (c) a rotary, pressure bonding process. The employed UCTAD paper towel had a basis weight of 40 g/m$^2$, and is available from Kimberly-Clark World Wide Sales, Inc., a business having offices located in Neenah, Wisconsin, U.S.A. The employed polymer film was a polyethylene-based film material, which was white in color, dimple embossed, and contained: 47.78% calcium carbonate, 2.22% TiO$_2$, and 50% polyethylene. The polymer film material was obtained from Pliant Corporation, a business having offices located in Chippewa Falls, Wisconsin, U.S.A. The strengths of the component web materials are summarized in the following Table 1.

**Table 1** -- Properties of webs

| Web material | Basis weight (g/m$^2$) | CD tensile Strength ± Stdv* (N/m) | MD tensile Strength ± Stdv* (N/m) |
|---|---|---|---|
| Film | 21.5 | 84.8 ± 6.6 | 583.6 ± 27.4 |
| Tissue | 40.0 | 268.9 ± 33.1 | 353.5 ± 22.7 |
| Note: * Stdv=standard deviation. | | | |

The high speed pressure bonding of the UCTAD paper towel and polymer film employed a JOA pressure bonder, which can be obtained from CURT G. JOA, Inc., a business having offices located in Sheboygan Falls, Wisconsin, U.S.A. The parameters of the bonder and the pin pattern used are listed in the following Table 2.

**Table 2** -- Bonder and pattern parameters:

| Bonder | | | Air cylinder | | | Pin pattern (circular shape pin) | | | | | | |
|---|---|---|---|---|---|---|---|---|---|---|---|---|
| $D_1$ mm | $D_2$ mm | Heating units | $D_3$ mm | $N_c$ | $N_s$ | $N_f$ | $N_l$ | $D_4$ mm | H mm | S mm | T mm | A mm |
| 190.5 | 190.5 | Yes* | 152.4 | 2 | 4 | 1 | 56 | 0.86 | 0.76 | 5.44 | 2.72 | 2.72 |

Note: The rollers can be heated to a temperature up to 200 °C from room temperature (~20°C).
$D_1$ - anvil roller diameter;
$D_2$ - pattern roller diameter;
$D_3$ - air cylinder diameter;
$N_c$ - number of air cylinders installed on the bonder;
$N_s$ - number of stages (pistons) of each air cylinder;
$N_f$ -number of pin pattern sleeves;
$N_l$ - number of pin lines of each pattern sleeve;
$D_4$ - pin diameter;
H - pin height;
S - pin interval in machine-direction;
T - pin interval in cross-direction;
A - pin offset in machine direction;
For the pin pattern used, the fraction (ratio) of pin area to pattern area is 0.039.

The bonded samples were tested. The bonding results and the corresponding bonding conditions are shown in the following Table 3.

**Table 3** -- Bonding conditions and results

| Cod e | Webs* | Nip Force (10$^6$ N/m) | Speed (m/sec) | Temp.** (°C) | Peel Bond Strength# (N/m) | Pin holes## % | Bonding Strength Observed |
|---|---|---|---|---|---|---|---|
| 1 | T/F | 1.66 | 1.02 | 54 | 8.81±1.43 | 0 | Good |
| 2 | T/F | 1.66 | 2.54 | 54 | 7.59±0.56 | 0 | Good |
| 3 | T/F | 1.66 | 4.06 | 54 | 6.86±1.40 | 0 | OK |
| 4 | T/F | 1.66 | 5.08 | 54 | 8.44±1.84 | 0 | Good |
| 5 | T/F | 2.49 | 1.02 | 54 | 9.15±0.38 | 0 | Good |
| 6 | T/F | 2.49 | 2.54 | 54 | 9.12±1.80 | 0 | Good |
| 7 | T/F | 2.49 | 4.06 | 54 | 7.95±0.51 | -10 | Overbond |
| 8 | T/F | 2.49 | 5.08 | 54 | 8.45±1.15 | 10-30 | Overbond |

(continued)

| Cod e | Webs* | Nip Force (10^6 N/m) | Speed (m/sec) | Temp.** (°C) | Peel Bond Strength# (N/m) | | Pin holes## % | Bonding Strength Observed |
|---|---|---|---|---|---|---|---|---|
| 9 | T/F | 3.32 | 1.02 | 54 | 9.28±2.97 | | 0 | Good |
| 10 | T/F | 3.32 | 2.54 | 54 | 9.83±2.15 | | <10 | Overbond |
| 11 | T/F | 3.32 | 4.06 | 54 | 8.29±1.30 | | 40-60 | Overbond |
| 12 | T/F | 3.32 | 5.08 | 54 | 8.43±1.64 | | 80-90 | Overbond |
| 13 | T/F/T | 1.66 | 1.02 | 54 | 7.32±2.53 | 6.35±1.86 | 0 | OK-Weak Bond |
| 14 | T/F/T | 1.66 | 2.54 | 54 | 8.56±2.48 | 6.51±1.96 | 0 | Good/ Weak Bond |
| 15 | T/F/T | 1.66 | 4.06 | 54 | 9.33±1.52 | 6.12±1.19 | 0 | Good /OK |
| 16 | T/F/T | 1.66 | 5.08 | 54 | 9.00±1.13 | 6.60±1.61 | 0 | Good /OK |
| 17 | T/F/T | 2.49 | 1.02 | 54 | 8.61±1.02 | 5.37±0.04 | 0 | Good/OK |
| 18 | T/F/T | 2.49 | 2.54 | 54 | 10.80±1.40 | 6.30±0.80 | <10 | Over Bond |
| 19 | T/F/T | 2.49 | 4.06 | 54 | 8.04±2.20 | 7.78±1.09 | 50-80 | Over Bond |
| 20 | T/F/T | 2.49 | 5.08 | 54 | 9.09±0.12 | 5.47±0.39 | -100 | Over Bond |
| 21 | T/F | 0.83 | 1.02 | 93 | 12.82±2.05 | | 0 | Good |
| 22 | T/F | 0.83 | 2.54 | 93 | 11.47±1.69 | | 0 | Good |
| 23 | T/F | 0.83 | 4.06 | 93 | 9.44±0.98 | | 0 | Good |
| 24 | T/F | 0.83 | 5.08 | 93 | 7.62±1.43 | | 0 | OK |
| 25 | T/F | 1.66 | 1.02 | 93 | 13.41±0.63 | | 0 | Good |
| 26 | T/F | 1.66 | 2.54 | 93 | 10.93±1.69 | | <10 | Over Bond |
| 27 | T/F | 1.66 | 4.06 | 93 | 9.69±4.44 | | 10-40 | Over Bond |
| 28 | T/F | 1.66 | 5.08 | 93 | 10.68±1.85 | | 60-90 | Over Bond |

Note: * T=paper towel, F=polymeric film;
** Temperature is pre-heated bonder temperature;
# Data errors shown in both positive and negative direction are one standard deviation;
## Pine holes appear when the webs are over-bonded under large nip forces.

With good bonding, the failure mode during the testing of the bond strength appeared to be a failure due to a tearing of the UCTAD material or polymer film, and the bonding strength was greater than 5 N/m. With low bonding, the bonding strength was less than 5 N/m; and during the testing of the bond strength, the UCTAD material delaminated from the polymer film without causing a failure of the UCTAD or polymer film materials. The designation "Holes" indicates that the laminate was overbonded. The laminate was damaged, and included an excessively large number of holes.

The following observations were made:

a) For tissue/film webs bonded at bonding speeds over 5 m/sec, the lowest bond strength was over 7 N/m; for tissue/film/tissue combinations, the lowest bond strength was over 5.5 N/m.

b) At high bonding speeds over 4 m/sec, the bond strength was not sensitive to the bonder temperature, indicating that a pre-heating or auxiliary heating during the bonder operation may not be necessary.

c) The nip force (e.g. lineal-pressure value) and bonder temperature can be adjusted to provide a combination which produces the desired bonding-strength and bond-formation. To obtain a given bond-strength, the nip force can be reduced if the bonder temperature is increased, and vice versa. If the combination of nip force and bonder temperature is too large, however, too much energy can be directed into the target web, and the bonded web material can be over-bonded (e.g., pin holes appear on the bonded webs).

For the materials considered in the examples, a suitable nip force range was from 1.5 MN/m to 3.0 MN/m, when the bonding was conducted at an ambient room temperature of 20-25 °C. The nip force needed can be reduced if the bonding operation includes a pre-heating or other auxiliary heating.

It was observed that the adhesively bonded laminates could be formed at high production speeds, but the absorbent intake rates and absorbent capacities of the adhesively bonded laminates were excessively low. The adhesively bonded laminates also showed an unfavorable, inflated surface due to a thermal shrinkage of the poly film caused by the heat absorbed from the melt-sprayed the adhesive. The thermal shrinkage caused an undesired, permanent curl in the final laminate sheet.

Compared to the adhesively laminated webs, the thermally bonded laminates exhibited improved absorbency. The thermally bonded webs, however, needed to be formed at a low machine speed. The low speed of the thermal bonding process was due to the low thermal conduction rate between the UCTAD paper towel layer and the polymer film layer. These low bonding speeds were inadequate for the desired production of large volumes of the bonded laminate sheet, and would excessively increase the capital and manufacturing costs.

It was found that a high speed rotary pressure bonding could more efficiently produce the desired laminate sheet. The bonding strength exhibited by the pressure-bonded web laminate was comparable to the bonding strength exhibited by the thermally bonded laminate, but the pressure-bonded web could exhibit the desired bonding strength even when produced at operating machine speeds of up to 5.1 m/sec.

Both single and dual absorbent side laminates were successfully prepared with the pressure-bonding process. The pressure-bonded laminate showed a much softer feel and a more attractive appearance, as compared to the thermal-embossed laminate and adhesive-bonded laminate. The high speed, rotary pressure-bonded web laminate could successfully incorporate two chemically incompatible web materials (cellulosic paper towel material with polymer film material). The bond-strength of the pressure-bonded laminate was comparable to the strengths exhibited by thermal-bonded webs or adhesive-bonded webs. In addition, the absorbency properties of the pressure-bonded laminate were approximately equal to or better than the absorbent properties of the paper base sheet (e.g. paper towel) alone. Information regarding the formed laminates are summarized in the following Table 4.

**Table 4**

| Sample | Materials | Absorbency | Method | Speed |
|--------|-----------|-----------|--------|-------|
| 1 | UCTAD only | 7.8 $\pm$ 0.5 g/g | Wet laid UCTAD | n/a |
| 2 | Single side laminate | 1 0.7 $\pm$ 0.5 g/g | High speed pressure lamination | 4.1 m/sec |
| 3 | Dual side laminate | 10.5 $\pm$ 0.5 g/g | High speed pressure lamination | 4.1 m/sec |
| 4 | Single side laminate | 9.4 $\pm$ 0.5 g/g | Thermal embossing, y-dot pattern | 0.1 m/sec |
| 5 | Single side laminate | 6.7 $\pm$ 0.5 g/g | Adhesive lamination; melt blown, sprayed at 1 g/m$^2$ add-on | 0.5 m/sec |
| 6 | Single side laminate | 7.2 $\pm$ 0.5 g/g | Adhesive lamination; melt blown swirl-sprayed at a 2 g/m$^2$ add-on | 0.5 m/sec |

Those skilled in the art will recognize that the present invention is capable of many modifications and variations without departing from the scope thereof. Accordingly, the detailed description and examples set forth above are meant to be illustrative only and are not intended to limit, in any manner, the scope of the invention as set forth in the appended claims.

**EP 1 937 458 B1**

**Claims**

1. A method of making a web article (28) comprising at least a first fibrous layer (36) laminated with a first facing surface (22) of at least one polymer film slayer (38); wherein the first fibrous layer (36) includes a first quantity of cellulosic fibers;

   the method comprising providing the at least one fibrous layer (36) and the at least one polymer film layer (38) configured to provide a target web (26);

   moving the target web (28) at a web speed of at least a minimum of 3.6 m/sec;

   moving the target web (28) at the web speed through a nip region (30) between a rotating anvil roller (34) and a counter-rotating pattern roller (32) to provide an operative first bond between the first fibrous layer (36) and the at least one film layer (38), wherein the anvil roller (34) and pattern roller (32) are urged together to provide a selected nip force value; and **characterised by** urging the anvil roller (34) and pattern roller (32) together to provide a lineal-pressure value of at least $0.05*10^6$ N/m.

2. A method as recited in claim 1, wherein the first bond between the first fibrous layer (36) and the at least one film layer (38) provides a bond peel-strength value of at least a minimum of 5 N/m.

3. A method as recited in claim 1, wherein the first fibrous layer (36) has a basis weight of fibrous material which is at least 10 g/m$^2$.

4. A method as recited in claim 1, wherein the web article (28) has a basis weight of polymer film (38) material which is at least 5 g/m$^2$, and the polymer film has a film thickness of at least 0.013 mm.

5. A method as recited in claim 1, wherein the article (28) has a fiber specific absorbent capacity of at least about 7 grams water per gram of the fibrous material in the fibrous layer of the article (28).

6. A method as recited in claim 1, wherein the first fibrous layer (36) includes a wet-laid tissue material.

7. A method as recited in claim 6, wherein the first fibrous layer (36) includes two or more plies of the wet-laid tissue material.

8. A method as recited in claim 1, wherein the first fibrous layer (36) includes an uncreped-through-air-dried layer of cellulosic, fibrous material; a pattern densified layer of cellulosic, fibrous material; or a cellulosic sheet material that includes a latex binder.

9. A method as recited in claim 8, wherein the first fibrous layer (36) comprises two or more plies of the uncreped-through-air-dried layer of cellulosic, fibrous material; the pattern densified layer of cellulosic, fibrous material; or the cellulosic sheet material.

10. A method as recited in claim 1 wherein the first fibrous layer (36) includes synthetic fibers.

11. A method as recited in claim 1, wherein the web article (28) further comprises a second fibrous layer (46) laminated with a second facing surface (74) of the at least one polymer film layer (38); wherein the second fibrous layer includes a second quantity of cellulosic fibers;

    the method comprising providing the target web (26) configured to include the second fibrous layer positioned on the second facing surface of the at least one polymer film layer; and

    moving the target web at the web speed through the nip region (30) between a rotating anvil roller (34) and a counter-rotating pattern roller (32) to provide an operative second bond between the second fibrous layer and the at least one film layer.

12. A method as recited in claim 11, wherein the second fibrous layer (46) has a basis weight within the range of at least 10 g/m$^2$.

13. A method as recited in claim 12, wherein the second fibrous layer (46) includes a wet-laid tissue material.

14. A method as recited in claim 12, wherein the second fibrous layer (46) includes two or more plies of a wet-laid material.

15. A method as recited in claim 12, wherein the second fibrous layer (46) includes an uncreped-through-air-dried layer of cellulosic material; a pattern densified layer of cellulosic, fibrous material; or a cellulosic sheet which includes a latex binder.

16. A method as recited in claim 12, wherein the second fibrous layer (46) includes two or more plies of uncreped-through-air-dried fibrous material.

17. A method as recited in claim 12, wherein the second bond between the second fibrous layer (46) and the at least one film layer (38) provides a bond peel-strength value of at least a minimum of 5 N/m.

18. A method as recited in claim 12, wherein the second fibrous layer (46) includes synthetic fibers.

19. A method as recited in claim 12, wherein the article (28) has a fiber specific absorbent capacity of at least 7 grams water per gram of the fibrous material in the fibrous layers of the article.

20. The method as claimed in any one of claims 1 or 4 to 11, wherein the first fibrous layer (36) is a tissue layer in facing relation with said at least one polymer film layer (38) to provide said target web (36).

21. The method of claim 20 wherein the deformation strain rate during the bonding compression of the target web (26) is within the range of $1*10^3$ to $1*10^4$ sec$^{-1}$.

22. The method of claim 20 comprising
providing the first tissue layer (36) in facing relation with the first facing surface (72) of the polymer film layer (38);
providing a second tissue layer (46) in facing relation with a second facing surface (74) of the polymer film layer, wherein the first tissue layer, the polymer film layer, and the second tissue layer provide the target web (26);
moving the target web through the nip region (30) between the rotating anvil roller (34) and the counter-rotating pattern roller (32) to provide an operative first bond between the first tissue layer and the polymer film layer and to provide an operative second bond between the second tissue layer and the polymer film layer, wherein the target web is moved at a web speed of at least 3.6 m/sec and wherein the anvil roller and pattern roller have been urged together to provide a selected nip force value and wherein the first bond between the first tissue layer and the film layer provides a bond peel-strength value of at least 5 N/m and wherein the second bond between the second tissue layer and the film layer provides a bond peel-strength value of at least 5 N/m.

23. The method of claim 22 wherein the second tissue layer (46) has a basis weight of at least 10 g/m$^2$.

24. The method of claim 23 wherein the second tissue layer (46) includes a wet-laid tissue material.

25. The method of claim 23 wherein the second tissue layer (46) includes two or more plies of a wet-laid material,

26. The method of claim 23 wherein the second tissue layer (46) includes an uncreped-through-air-dried layer of cellulosic material; a pattern densified layer of cellulosic, fibrous material; or a cellulosic sheet which includes a latex binder.

27. The method of claim 23 wherein the second tissue layer (46) includes two or more plies of uncreped-through-air-dried fibrous material,

28. The method of claim 23 wherein the second tissue layer (46) includes synthetic fibers.

29. The method of claim 23 wherein the web article (28) has a fiber specific absorbent capacity of at least 7 grams water per gram of the tissue material in the first and second tissue layers (36,46) of the article (28).

30. The method of claim 22 wherein the deformation strain rate during the bonding compression of the target web (26) is within the range of $1*10^3$ to $1*10^4$ sec$^{-1}$.


**Patentansprüche**

1. Verfahren zum Herstellen eines bahnförmigen Artikels (28), der wenigstens eine faserartige Lage (36) umfasst, die mit einer ersten Sichtoberfläche (22) aus wenigstens einer Polymerfilmlage (38) beschichtet ist; wobei die erste faserartige Lage (36) eine erste Menge von Zellulosefasern enthält;

wobei das Verfahren Folgendes umfasst: Bereitstellen der wenigstens einen faserartigen Lage (36) und der wenigstens einen Polymerfilmlage (38), die eingerichtet sind, um eine gewünschte Bahn (26) zu bilden;
Bewegen der gewünschten Bahn (28) mit einer Bahngeschwindigkeit von wenigstens 3,6 m/s;
Bewegen der gewünschten Bahn (28) mit der Bahngeschwindigkeit durch einen Druckbereich (30) zwischen einer rotierenden Ambosswalze (34) und einer entgegengesetzt rotierenden Musterwalze (32), um eine funktionsfähige erste Bindung zwischen der ersten faserartigen Lage (36) und der wenigstens einen Filmlage (38) zu schaffen, wobei die Ambosswalze (34) und die Musterwalze (32) gegeneinander gedrängt werden, um einen ausgewählten Druckkraftwert zu erzeugen; und **gekennzeichnet durch** Drängen der Ambosswalze (34) und der Musterwalze (32) gegeneinander, um einen Linealdruckwert von wenigstens 0,05 *$10^6$ N/m zu erzeugen.

2. Verfahren nach Anspruch 1, wobei die erste Bindung zwischen der ersten faserartigen Lage (36) und der wenigstens einen Filmlage (38) einen Wert der Bindungsabschälfestigkeit von wenigstens 5 N/m erzeugt.

3. Verfahren nach Anspruch 1, wobei die erste faserartige Lage (36) ein Basisgewicht des faserartigen Werkstoffs aufweist, das wenigstens 10 g/m$^2$ beträgt.

4. Verfahren nach Anspruch 1, wobei der bahnförmige Artikel (28) ein Basisgewicht des Polymerfilms (38) aufweist, das wenigstens 5 g/m$^2$ beträgt, und der Polymerfilm eine Filmdicke von wenigstens 0,013 mm aufweist.

5. Verfahren nach Anspruch 1, wobei der Artikel (28) eine faserspezifische Absorptionsfähigkeit des faserartigen Werkstoffs in der faserartigen Lage des Artikels (28) von wenigstens etwa 7 Gramm Wasser pro Gramm aufweist.

6. Verfahren nach Anspruch 1, wobei die erste faserartige Lage (36) ein nassgelegtes Gewebematerial enthält.

7. Verfahren nach Anspruch 6, wobei die erste faserartige Lage (36) zwei oder mehr Stofflagen des nassgelegten Gewebematerials enthält.

8. Verfahren nach Anspruch 1, wobei die erste faserartige Lage (36) eine nicht gekreppte luftgetrocknete Lage des faserartigen Zellulosematerials, eine durch ein Muster verdichtete Lage des faserartigen Zellulosematerials oder ein ein Latexbindemittel enthaltendes Zellulosebahnmaterial enthält.

9. Verfahren nach Anspruch 8, wobei die erste faserartige Lage (36) zwei oder mehr Stofflagen der nicht gekreppten luftgetrockneten Lage des faserartigen Zellulosematerials, der durch ein Muster verdichteten Lage des faserartigen Zellulosematerials oder des Zellulosebahnmaterials enthält.

10. Verfahren nach Anspruch 1, wobei die erste faserartige Lage (36) synthetische Fasern enthält.

11. Verfahren nach Anspruch 1, wobei der bahnförmige Artikel (28) ferner eine zweite faserartige Lage (46), die mit einer zweiten Sichtoberfläche (74) der wenigstens einen Polymerfilmlage (38) beschichtet ist, umfasst; wobei die zweite faserartige Lage eine zweite Menge der Zellulosefasern enthält;
wobei das Verfahren Folgendes umfasst: Bereitstellen der gewünschten Bahn (26), die eingerichtet ist, um die zweite faserartige Lage zu enthalten, die auf der zweiten Sichtoberfläche der wenigstens einen Polymerfilmlage positioniert ist; und
Bewegen der gewünschten Bahn mit der Bahngeschwindigkeit durch den Druckbereich (30) zwischen einer rotierenden Ambosswalze (34) und einer entgegengesetzt rotierenden Musterwalze (32), um eine funktionsfähige zweite Bindung zwischen der zweiten faserartigen Lage und der wenigstens einen Filmlage zu schaffen.

12. Verfahren nach Anspruch 11, wobei die zweite faserartige Lage (46) ein Basisgewicht in dem Bereich von wenigstens 10 g/m$^2$ aufweist.

13. Verfahren nach Anspruch 12, wobei die zweite faserartige Lage (46) ein nassgelegtes Gewebematerial enthält.

14. Verfahren nach Anspruch 12, wobei die zweite faserartige Lage (46) zwei oder mehr Stofflagen eines nassgelegten Gewebematerials enthält.

15. Verfahren nach Anspruch 12, wobei die zweite faserartige Lage (46) eine nicht gekreppte luftgetrocknete Lage des faserartigen Zellulosematerials, eine durch ein Muster verdichtete Lage des faserartigen Zellulosematerials oder eine ein Latexbindemittel enthaltende Zellulosebahn enthält.

16. Verfahren nach Anspruch 12, wobei die zweite faserartige Lage (46) eine oder mehr Stofflagen eines nicht gekreppten luftgetrockneten faserartigen Materials enthält.

17. Verfahren nach Anspruch 12, wobei die zweite Bindung zwischen der zweiten faserartigen Lage (46) und der wenigstens einen Filmlage (38) einen Wert der Bindungsabschälfestigkeit von wenigstens 5 N/m erzeugt.

18. Verfahren nach Anspruch 12, wobei die zweite faserartige Lage (46) synthetische Fasern enthält.

19. Verfahren nach Anspruch 12, wobei der Artikel (28) eine faserspezifische Absorptionsfähigkeit des faserartigen Materials in der faserartigen Lage des Artikels von wenigstens 7 Gramm Wasser pro Gramm aufweist.

20. Verfahren nach einem der Ansprüche 1 oder 4 bis 11, wobei die erste faserartige Lage (36) eine Gewebelage ist, die der wenigstens einen Polymerfilmlage (38) zugewandt ist, um die gewünschte Bahn (36) zu erzeugen.

21. Verfahren nach Anspruch 20, wobei die Verformungsspannungsrate während der Bindungskompression der gewünschten Bahn (26) im Bereich von $1 * 10^3$ bis $1 * 10^4$ $s^{-1}$ liegt.

22. Verfahren nach Anspruch 20, das Folgendes umfasst:

Bereitstellen einer ersten Gewebelage (36), die der ersten Sichtoberfläche (72) der Polymerfilmlage (38) zugewandt ist;
Bereitstellen einer zweiten Gewebelage (46), die der zweiten Sichtoberfläche (74) der Polymerfilmlage zugewandt ist, wobei die erste Gewebelage, die zweite Polymerfilmlage und die zweite Gewebelage die gewünschte Bahn (26) erzeugen;
Bewegen der gewünschten Bahn durch den Druckbereich (30) zwischen der rotierenden Ambosswalze (34) und der entgegengesetzt rotierenden Musterwalze (32), um eine funktionsfähige erste Bindung zwischen der ersten Gewebelage und der Polymerfilmlage zu schaffen und eine funktionsfähige zweite Bindung zwischen der zweiten Gewebelage und der Polymerfilmlage zu schaffen, wobei die gewünschte Bahn mit einer Bahngeschwindigkeit von wenigstens 3,6 m/s bewegt wird und wobei die Ambosswalze und die Musterwalze zusammengedrückt werden, um einen ausgewählten Druckkraftwert zu erzeugen und wobei die erste Bindung zwischen der ersten Gewebelage und der Filmlage einen Wert der Bindungsabschälfestigkeit von wenigstens 5 N/m erzeugt und wobei die zweite Bindung zwischen der zweiten Gewebelage und der Filmlage einen Wert der Bindungsabschälfestigkeit von wenigstens 5 N/m erzeugt.

23. Verfahren nach Anspruch 22, wobei die zweite Gewebelage (46) ein Basisgewicht von wenigstens 10 $g/m^2$ aufweist.

24. Verfahren nach Anspruch 23, wobei die zweite Gewebelage (46) ein nassgelegtes Gewebematerial enthält.

25. Verfahren nach Anspruch 23, wobei die zweite Gewebelage (46) zwei oder mehr Stofflagen eines nassgelegten Materials enthält.

26. Verfahren nach Anspruch 23, wobei die zweite Gewebelage (46) eine nicht gekreppte luftgetrocknete Lage des Zellulosematerials, eine durch ein Muster verdichtete Lage des faserartigen Zellulosematerials oder eine ein Latexbindemittel enthaltende Zellulosebahn enthält.

27. Verfahren nach Anspruch 23, wobei die zweite Gewebelage (46) zwei oder mehr Stofflagen des nicht gekreppten luftgetrockneten faserartigen Materials enthält.

28. Verfahren nach Anspruch 23, wobei die zweite Gewebelage (46) synthetische Fasern enthält.

29. Verfahren nach Anspruch 23, wobei der bahnförmige Artikel (28) eine faserspezifische Absorptionsfähigkeit des Gewebematerials in den ersten und zweiten Gewebelagen (36, 46) des Artikels (28) von wenigstens 7 Gramm Wasser pro Gramm aufweist.

30. Verfahren nach Anspruch 22, wobei die Verformungsspannungsrate während der Bindungskompression der gewünschten Bahn (26) im Bereich von $1 * 10^3$ bis $1 * 10^4$ $s^{-1}$ liegt.

**Revendications**

1. Procédé de fabrication d'un article en voile (28) comprenant au moins une première couche fibreuse (36) stratifiée avec une première face opposée (22) d'au moins une couche de film polymère (38) ; dans lequel la première couche fibreuse (36) comporte une première quantité de fibres cellulosiques ;
le procédé comprenant la fourniture de la au moins une couche fibreuse (36) et de la au moins une couche de film polymère (38) configurées pour fournir un voile cible (26) ;
le déplacement du voile cible (28) à une vitesse de voile d'au moins un minimum de 3,6 m/s ;
le déplacement du voile cible (28) à la vitesse de voile à travers une région de contact (30) entre un contre-cylindre rotatif (34) et un cylindre à motif, contrarotatif (32) pour fournir un premier assemblage opérationnel entre la première couche fibreuse (36) et la au moins une couche de film (38), dans lequel le contre-cylindre (34) et le cylindre à motif (32) sont poussés ensemble pour fournir une valeur de force de contact sélectionnée ; et
**caractérisé par** la poussée conjointe du contre-cylindre (34) et du cylindre à motif (32) pour fournir une valeur de pression linéaire d'au moins $0,05*10^6$ N/m.

2. Procédé tel qu'exposé dans la revendication 1, dans lequel le premier assemblage entre la première couche fibreuse (36) et la au moins une couche de film (38) fournit une valeur de résistance à l'arrachement de l'assemblage d'au moins un minimum de 5 N/m.

3. Procédé tel qu'exposé dans la revendication 1, dans lequel la première couche fibreuse (36) a un poids surfacique de matière fibreuse qui est d'au moins 10 g/m$^2$.

4. Procédé tel qu'exposé dans la revendication 1, dans lequel l'article en voile (38) a un poids surfacique de film polymère (28) qui est d'au moins 5 g/m$^2$, et le film polymère a une épaisseur de film d'au moins 0,013 mm.

5. Procédé tel qu'exposé dans la revendication 1, dans lequel l'article (28) a une capacité d'absorption spécifique des fibres d'au moins environ 7 grammes d'eau par gramme de la matière fibreuse dans la couche fibreuse de l'article (28).

6. Procédé tel qu'exposé dans la revendication 1, dans lequel la première couche fibreuse (36) comporte une matière de tissu posée humide.

7. Procédé tel qu'exposé dans la revendication 6, dans lequel la première couche fibreuse (36) comporte deux ou plus de deux plis de la matière de tissu posée humide.

8. Procédé tel qu'exposé dans la revendication 1, dans lequel la première couche fibreuse (36) comporte une couche de matière fibreuse, cellulosique, non crêpée séchée par soufflage d'air transversal ; une couche de matière fibreuse, cellulosique, densifiée, à motif ; ou une matière en feuille cellulosique qui comporte un liant au latex.

9. Procédé tel qu'exposé dans la revendication 8, dans lequel la première couche fibreuse (36) comprend deux ou plus de deux plis de la couche de matière fibreuse, cellulosique, non crêpée séchée par soufflage d'air transversal ; de la couche de matière fibreuse, cellulosique, densifiée, à motif ; ou de la matière en feuille cellulosique.

10. Procédé tel qu'exposé dans la revendication 1, dans lequel la première couche fibreuse (36) comporte des fibres synthétiques.

11. Procédé tel qu'exposé dans la revendication 1, dans lequel l'article en voile (28) comprend en outre une seconde couche fibreuse (46) stratifiée avec une seconde surface opposée (74) de la au moins une couche de film polymère (38) ; dans lequel la seconde couche fibreuse comporte une seconde quantité de fibres cellulosiques ;
le procédé comprenant la fourniture du voile cible (26) configuré pour comporter la seconde couche fibreuse positionnée sur la seconde face opposée de la au moins une couche de film polymère ; et
le déplacement du voile cible à la vitesse de voile à travers la région de contact (30) entre un contre-cylindre rotatif (34) et un cylindre à motif contrarotatif (32) pour fournir un second assemblage opérationnel entre la seconde couche fibreuse et la au moins une couche de film.

12. Procédé tel qu'exposé dans la revendication 11, dans lequel la seconde couche fibreuse (46) a un poids surfacique dans l'intervalle d'au moins 10 g/m$^2$.

13. Procédé tel qu'exposé dans la revendication 12, dans lequel la seconde couche fibreuse (46) comporte une matière de tissu posée humide.

**14.** Procédé tel qu'exposé dans la revendication 12, dans lequel la seconde couche fibreuse (46) comporte deux ou plus de deux plis de matière posée humide.

**15.** Procédé tel qu'exposé dans la revendication 12, dans lequel la seconde couche fibreuse (46) comporte une couche de matière cellulosique non crêpée séchée par soufflage d'air transversal ; une couche de matière fibreuse, cellulosique, densifiée, à motif ; ou une feuille cellulosique qui comporte un liant au latex.

**16.** Procédé tel qu'exposé dans la revendication 12, dans lequel la seconde couche fibreuse (46) comporte deux ou plus de deux plis de matière fibreuse non crêpée séchée par soufflage d'air transversal.

**17.** Procédé tel qu'exposé dans la revendication 12, dans lequel le second assemblage entre la seconde couche fibreuse (46) et la au moins une couche de film (38) fournit une valeur de résistance à l'arrachement de l'assemblage d'au moins un minimum de 5 N/m.

**18.** Procédé tel qu'exposé dans la revendication 12, dans lequel la seconde couche fibreuse (46) comporte des fibres synthétiques.

**19.** Procédé tel qu'exposé dans la revendication 12, dans lequel l'article (28) a une capacité d'absorption spécifique des fibres d'au moins 7 grammes d'eau par gramme de la matière fibreuse dans les couches fibreuses de l'article.

**20.** Procédé tel que revendiqué dans l'une quelconque des revendications 1 ou 4 à 11, dans lequel la première couche fibreuse (36) est une couche de tissu en relation opposée avec ladite au moins une couche de film polymère (38) pour fournir ledit voile cible (36).

**21.** Procédé selon la revendication 20, dans lequel la vitesse de déformation pendant la compression d'assemblage du voile cible (26) est dans l'intervalle de $1*10^3$ à $1*10^4$ s$^{-1}$.

**22.** Procédé selon la revendication 20, comprenant
la fourniture de la première couche de tissu (36) en relation opposée avec la première surface opposée (72) de la couche de film polymère (38) ;
la fourniture d'une seconde couche de tissu (46) en relation opposée avec une seconde surface opposée (74) de la couche de film polymère, dans lequel la première couche de tissu, la couche de film polymère et la seconde couche de tissu fournissent le voile cible (26) ;
le déplacement du voile cible à travers la région de contact (30) entre le contre-cylindre rotatif (34) et le cylindre à motif, contrarotatif (32) pour fournir un premier assemblage opérationnel entre la première couche de tissu et la couche de film polymère et pour fournir un second assemblage opérationnel entre la seconde couche de tissu et la couche de film polymère ; dans lequel le voile cible est déplacé à une vitesse de voile d'au moins 3,6 m/s et dans lequel le contre-cylindre et le cylindre à motif ont été poussés ensemble pour fournir une valeur de force de contact sélectionnée et dans lequel le premier assemblage entre la première couche de tissu et la couche de film fournit une valeur de résistance à l'arrachement de l'assemblage d'au moins 5 N/m et dans lequel le second assemblage entre la seconde couche de tissu et la couche de film fournit une valeur de résistance à l'arrachement de l'assemblage d'au moins 5 N/m.

**23.** Procédé selon la revendication 22, dans lequel la seconde couche de tissu (46) a un poids surfacique d'au moins 10 g/m$^2$.

**24.** Procédé selon la revendication 23, dans lequel la seconde couche de tissu (46) comporte une matière de tissu posée humide.

**25.** Procédé selon la revendication 23, dans lequel la seconde couche de tissu (46) comporte deux ou plus de deux plis de matière posée humide.

**26.** Procédé selon la revendication 23, dans lequel la seconde couche de tissu (46) comporte une couche de matière cellulosique non crêpée séchée par soufflage d'air transversal ; une couche de matière fibreuse, cellulosique, densifiée, à motif ; ou une feuille cellulosique qui comporte un liant au latex.

**27.** Procédé selon la revendication 23, dans lequel la seconde couche de tissu (46) comporte deux ou plus de deux plis de matière fibreuse non crêpée séchée par soufflage d'air transversal.

**28.** Procédé selon la revendication 23, dans lequel la seconde couche de tissu (46) comporte des fibres synthétiques.

**29.** Procédé selon la revendication 23, dans lequel l'article en voile (28) a une capacité d'absorption spécifique des fibres d'au moins 7 grammes d'eau par gramme de la matière de tissu dans les première et seconde couches de tissu (36, 46) de l'article (28).

**30.** Procédé selon la revendication 22, dans lequel la vitesse de déformation pendant la compression d'assemblage du voile cible (26) est dans l'intervalle de $1*10^3$ à $1*10^4$ s$^{-1}$.

**FIG. 1**

**FIG. 1A**

FIG. 2

**FIG. 2A**

**FIG. 2B**

**EP 1 937 458 B1**

**Patent documents cited in the description**

- WO 9828123 A
- WO 0009314 A
- US 3879257 A, Gentile
- US 5399412 A, S, J. Sudall and S. A. Engel
- US 5672248 A, Wendt
- US 5656132 A
- US 6120642 A
- US 6096169 A
- US 6197154 B
- US 6143135 A
- US 6808790 B2
- US 4514345 A, Johnson
- US 4528239 A, Trokhan
- US 5098522 A
- US 5275700 A, Trokhan
- US 5328565 A, Rasch
- US 5334289 A, Trokhan
- US 5431786 A, Rasch
- US 5496624 A, Steltjes, Jr.
- US 5624790ISSUED A, Trokhan
- US 5628876 A, Ayers
- US 19278102 A
- US 10447321 A
- US 10326915 A
- US 10382222 A
- US 10319415 A
- US 10749475 A
- US 20060266473 A